(19) Europäisches Patentamt / European Patent Office / Office européen des brevets

(11) **EP 4 161 450 B1**

(12) **EUROPEAN PATENT SPECIFICATION**

(45) Date of publication and mention
of the grant of the patent:
**16.07.2025  Bulletin 2025/29**

(21) Application number: **21739780.1**

(22) Date of filing: **06.06.2021**

(51) International Patent Classification (IPC):
*A61F 2/44* *(2006.01)*        *A61F 2/30* *(2006.01)*

(52) Cooperative Patent Classification (CPC):
**A61F 2/4455;** A61F 2002/30014; A61F 2002/4495

(86) International application number:
**PCT/IB2021/054947**

(87) International publication number:
**WO 2021/245633 (09.12.2021 Gazette 2021/49)**

(54) **DYNAMIC INTERBODY FUSION DEVICES**

DYNAMISCHE ZWISCHENWIRBELFUSIONSVORRICHTUNGEN

DISPOSITIFS DE FUSION INTERSOMATIQUE DYNAMIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **05.06.2020  US 202063035262 P**

(43) Date of publication of application:
**12.04.2023  Bulletin 2023/15**

(73) Proprietor: **NuVasive, Inc.**
**San Diego, CA 92121 (US)**

(72) Inventors:
• **CHEVALIER, Éric**
**62000 Arras (FR)**
• **KERSPERN, Valentin**
**92320 Châtillon (FR)**

(74) Representative: **Morabito, Sara**
**Cantaluppi & Partners S.r.l.**
**Piazzetta Cappellato Pedrocchi, 18**
**35122 Padova (IT)**

(56) References cited:
**WO-A1-2004/017857       US-A1- 2018 280 145**
**US-A1- 2019 133 783       US-A1- 2020 046 512**

## Description

### FIELD OF DISCLOSURE

The present disclosure provides dynamic interbody fusion devices and methods of making and using same.

### BACKGROUND

Patients experiencing spinal diseases or disorders such as degenerative disc disease, or spinal injury often require placement of an interbody cage between two adjacent vertebrae. Non-union of the adjacent vertebrae remains a persistent complication, however. Prior art devices have improved procedures required to implant the interbody cage, but require relatively stiff interbody cages to minimize stress shielding of ingrown bone and reduce the risk of cage subsidence. The stiff nature of these interbody cages generally negates any potentially beneficial effects of vibrational waves and/or graft strain on the bone growth process.

US2018/0280145 describes a three-dimensional lattice structure for use in medical implants.

There remains a need for improved spinal implants that modify (e.g., enable, enhance, redirect, control, and/or dampen) applied mechanical load (e.g., a compression force, a torsion force, a sheer force, or a vibrational wave). The present disclosure describes systems and methods (not claimed) that meet those needs.

### SUMMARY

The present invention provides a dynamic spinal implant configured to be inserted between two adjacent vertebrae, as set out in claim 1.

The present disclosure provides a dynamic spinal implant configured to be inserted between two adjacent vertebrae, the device comprising: a first endplate configured to contact the inferior face of a first vertebrae; a second endplate substantially opposite the first endplate and configured to contact the superior face of a second, adjacent vertebrae; and an inner structure disposed between the first endplate and the second endplate, wherein the inner structure is configured to generate a modified strain tensor on bone graft and/or bone substitute material disposed within the dynamic spinal implant

The present disclosure describes a method of promoting intervertebral bone growth in a subject, the method comprising: inserting a dynamic interbody fusion device of any one preceding claim between two adjacent vertebrae; and optionally applying a mechanical load to the dynamic interbody fusion device for a period of time sufficient to promote bone growth.

These and other features are described in greater detail herein.

### BRIEF DESCRIPTION OF THE DRAWINGS

FIG. 1 shows a perspective view of a dynamic interbody fusion device with an anterior plate inserted between two adjacent vertebrae consistent with one embodiment of the present disclosure.

FIG. 2A shows a perspective view of a dynamic interbody fusion device consistent with one embodiment of the present disclosure.

FIG. 2B shows a plan view of an anterior plate consistent with one embodiment of the present disclosure.

FIG. 3A shows a perspective transparent view of a dynamic interbody fusion device consistent with another embodiment of the present disclosure.

FIG. 3B shows a side plan transparent view of the dynamic interbody fusion device of FIG. 3A. FIG. 3C shows a top plan transparent view of the dynamic interbody fusion device of FIG. 3A.

FIG. 4 shows a perspective view of an orthorhombic body-centered repeatable unit of an inner structure of a dynamic interbody fusion device consistent with one embodiment of the present disclosure.

FIG. 5 shows a perspective view of a caged orthorhombic body-centered repeatable unit of an inner structure of a dynamic interbody fusion device consistent with one embodiment of the present disclosure.

FIG. 6 shows a perspective view of a dode-thin lattice repeatable unit of an inner structure of a dynamic interbody fusion device consistent with one embodiment of the present disclosure.

FIG. 7 shows a perspective view of a diamond repeatable unit of an inner structure of a dynamic interbody fusion device consistent with one embodiment of the present disclosure.

FIG. 8A shows a perspective view of an auxetic repeatable unit of an inner structure of a dynamic interbody fusion device consistent with one embodiment of the present disclosure.

FIG. 8B shows a side plan view of the auxetic repeatable unit of the inner structure of the dynamic interbody fusion device of FIG. 8A.

FIG. 9 shows a perspective view of an S-shaped repeatable unit of an inner structure of a dynamic interbody fusion device consistent with one embodiment of the present disclosure.

FIG. 10 shows a resonating element suitable for incorporation into an inner structure of a dynamic interbody fusion device consistent with one embodiment of the present disclosure.

FIG. 11 shows a resonating element suitable for incorporation into an inner structure of a dynamic interbody fusion device consistent with another embodiment of the present disclosure.

FIG. 12 shows a free resonator suitable for incorporation into an inner structure of a dynamic interbody fusion device consistent with one embodiment of the present disclosure.

FIG. 13 shows a perspective view of an auxetic repeatable unit including one incorporated resonating element consistent with one embodiment of the present disclosure.

FIG. 14 shows a perspective view of an auxetic repeatable unit including five incorporated resonating elements each having a unique axis of motion consistent with one embodiment of the present disclosure.

FIG. 15 shows a perspective view of an orthorhombic body-centered repeatable unit including multiple tuned mass resonating elements consistent with one embodiment of the present disclosure.

FIG. 16 shows a perspective view of a caged orthorhombic body-centered repeatable unit including a free resonator consistent with one embodiment of the present disclosure.

FIG. 17 shows a perspective view of a mechanical load director suitable for incorporation into a dynamic interbody fusion device consistent with one embodiment of the present disclosure.

FIG. 18 shows a perspective view of a mechanical load director suitable for incorporation into a dynamic interbody fusion device consistent with another embodiment of the present disclosure.

FIG. 19A shows variation in the stiffness of a controlled-rigidity type dynamic interbody fusion device along the x- and z-axes consistent with one embodiment of the present disclosure.

FIG. 19B shows variation in the stiffness of a controlled low-rigidity auxetic lattice type dynamic interbody fusion device along the x- and z-axes consistent with one embodiment of the present disclosure.

FIG. 20 shows a cross-sectional view of a dynamic interbody fusion device including an internal graft material consistent with one embodiment of the present disclosure.

FIG. 21A shows a transversal cross-sectional view of a dynamic interbody fusion device including a graft shield component that is isolated from the outer cage portion of the device consistent with one embodiment of the present disclosure.

FIG. 21B shows a front cross-sectional view of the dynamic interbody fusion device of FIG. 21A.

FIG. 22 shows an anterior plan view of a dynamic interbody fusion device including a slit disposed between cranial fixation port(s) and caudal fixation port(s) consistent with one embodiment of the present disclosure.

FIG. 23A shows a transversal cross-sectional view of a dynamic interbody fusion device including a plurality of resonators configured to move freely along the caudo-cranial axis of the device.

FIG. 23B shows an enlarged view of one type of free resonators suitable for inclusion in the device of FIG. 23A.

FIG. 23C shows an enlarged view of another type of free resonators suitable for inclusion in the device of FIG. 23A.

FIG. 24A shows a transversal cross-sectional view of a dynamic interbody fusion device including a cavity that is isolated from external loads via an interface connected to the endplate via one or more springs, consistent with one embodiment of the present disclosure.

FIG. 24B shows an enlarged lateral cutaway view of the dynamic interbody fusion device of FIG. 24A.

FIG. 25A shows a lateral cutaway perspective view of a dynamic interbody fusion device including a plurality of resonating elements disposed in the graft cavity.

FIG. 25B shows a lateral cutaway perspective view of a dynamic interbody fusion device including a plurality of free resonators disposed in the graft cavity.

FIG. 26 shows an interbody cage portion consistent with the present disclosure and studied *in silico* according to Examples 1-2.

FIG. 27 shows dynamic response to the *in silico* forced excitation of the interbody cage of FIG. 26 according to Example 1.

FIG. 28 shows anatomical parameters utilized in the *in silico* study of the interbody cage portion of FIG. 26 in Example 2.

FIGS. 29A-29C show observed Von Mises stresses in the cage, inner structure/lattice and graft shield (FIG. 29A), in the cortical bone (FIG. 29B), and in the trabecular bone (FIG. 29C) in the *in silico* study of Example 2.

FIG. 30 shows the dynamic response of the graft shield during imposed displacement of the S1 vertebrae in the *in silico* study of Example 2.

FIG. 31 shows the graft shield dynamic response as a function of position over time in the *in silico* study of Example 2.

FIG. 32 shows the graft shield dynamic response as a function of velocity over time in the *in silico* study of Example 2.

FIG. 33 shows a fine element model of a free resonator system generated for *in silico* study consistent with Example 3.

FIG. 34A shows vibrational modes observed when the fine element model of FIG. 33 using soft graft theoretical material was subjected to forced vertical excitation of the horizontal boundaries at 30 Hz and at 0.4 mm amplitude.

FIG. 34B shows vibrational modes observed when the fine element model of FIG. 33 using soft graft theoretical material was subjected to forced vertical excitation of the horizontal boundaries at 90 Hz and at 0.4 mm amplitude.

FIG. 34C shows vibrational modes observed when the fine element model of FIG. 33 using normal graft theoretical material was subjected to forced vertical excitation of the horizontal boundaries at 90 Hz and at 0.4 mm amplitude.

FIG. 35A shows displacement of three resonating elements of the fine element model of FIG. 33 using soft graft theoretical material was subjected to forced vertical excitation of the horizontal boundaries at 30 Hz and at 0.4 mm amplitude.

FIG. 35B shows displacement of three resonating elements of the fine element model of FIG. 33 using soft graft theoretical material was subjected to forced vertical excitation of the horizontal boundaries at 90 Hz and at 0.4 mm amplitude.

FIG. 35C shows displacement of three resonating elements of the fine element model of FIG. 33 using normal graft theoretical material was subjected to forced vertical excitation of the horizontal boundaries at 90 Hz and at 0.4 mm amplitude.

FIG. 36A shows observed vibrational modes when the fine element model of FIG. 33 sing soft graft theoretical material was subjected to forced vertical excitation of the horizontal boundaries at 30 Hz and at 0.4 mm amplitude.

FIG. 36B shows observed vibrational modes when the fine element model of FIG. 33 sing soft graft theoretical material was subjected to forced vertical excitation of the horizontal boundaries at 90 Hz and at 0.4 mm amplitude.

FIG. 37A shows a front plan view of a dynamic interbody fusion device not including endplate structures consistent with one embodiment of the present disclosure.

FIG. 37B shows a top plan view of the dynamic interbody fusion device of FIG. 37A.

FIG. 38A shows a front plan view of a dynamic interbody fusion device including a slit (e.g., continuous slit) around its perimeter consistent with one embodiment of the present disclosure.

FIG. 38B shows a side plan view of the dynamic interbody fusion device of FIG. 38A.

FIG. 38C shows a rear plan view of the dynamic interbody fusion device of FIG. 38A.

FIG. 38D shows a top plan view of the dynamic interbody fusion device of FIG. 38A.

FIG. 38E shows a first cross-sectional view of the dynamic interbody fusion device of FIG. 38A.

FIG. 38F shows a second cross-sectional view of the dynamic interbody fusion device of FIG. 38A.

FIG. 38G shows a third cross-sectional view of the dynamic interbody fusion device of FIG. 38A.

FIG. 38H shows an enlarged sectional view of the dynamic interbody fusion device of FIG. 38A.

FIG. 38I shows a front photographic view of the dynamic interbody fusion device of FIG. 38A.

FIG. 38J shows a side photographic view of the dynamic interbody fusion device of FIG. 38A.

FIG. 38K shows a rear photographic view of the dynamic interbody fusion device of FIG. 38A.

FIG. 38L shows a top photographic view of the dynamic interbody fusion device of FIG. 38A.

FIG. 39 shows a perspective view of a mechanical load director suitable for incorporation into a dynamic interbody fusion device consistent with another embodiment of the present disclosure.

FIG. 40 shows a schematic view of an *in silico* compression load test performed on dynamic interbody fusion devices consistent with the present disclosure.

FIG. 41A shows a dynamic interbody fusion device consistent with one embodiment of the present disclosure.

FIG. 41B shows a dynamic interbody fusion device consistent with another embodiment of the present disclosure.

FIG. 41C shows comparative results from the *in silico* compression load test of FIG. 40 on the dynamic interbody fusion devices of FIGS. 41A and 41B.

FIG. 42 is a photograph showing (clockwise from top center): a prototype dynamic interbody fusion device consistent with the present disclosure, prototypes of two portions of inner structures including an auxetic repeatable unit pattern, and prototypes of two portion of inner structures each including five free resonator elements.

The figures depict various embodiments of this disclosure for purposes of illustration only. One skilled in the art will readily recognize from the following discussion that alternative embodiments of the structures and methods illustrated herein may be employed without departing from the principles of embodiments described herein.

**DETAILED DESCRIPTION**

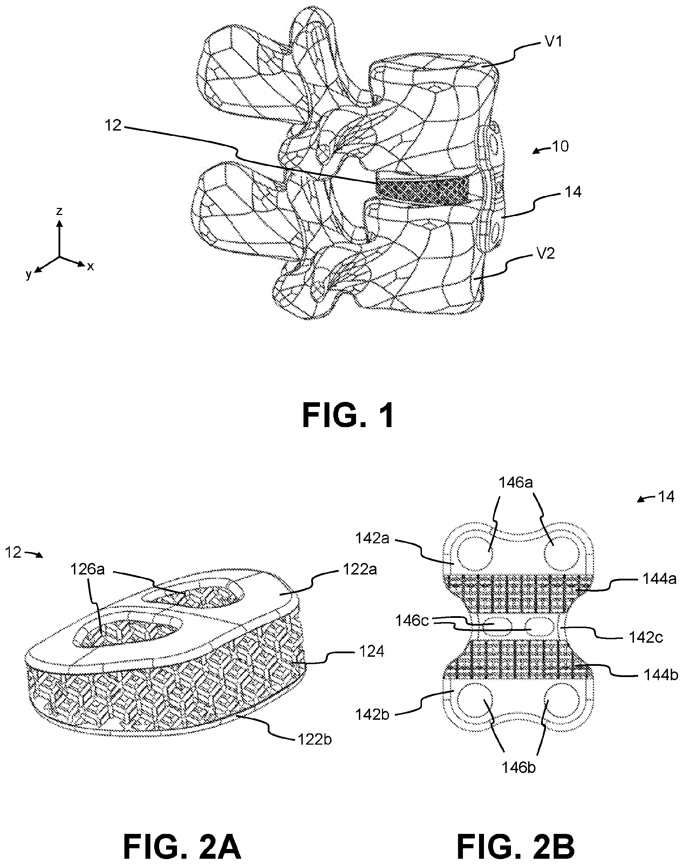

Referring generally to FIGS. 1-42, the present disclosure provides dynamic interbody fusion devices; and inner structure repeatable units, resonating elements, free resonators, and mechanical load directors for incorporation therein.

1. Dynamic Interbody Fusion Devices

Generally, spinal implant systems consistent with the present disclosure are intended to fuse together two adjacent vertebrae. The spinal implant systems can be an interbody fusion cage, an anterior plate, a posterior fixation, or a combination of those.

In some embodiments, the spinal implant comprises load sharing and deformable structure design allowing graft strain in a desired range (e.g., +/-15%).

The strain experienced by the graft is a direct result of the spinal implant deformation. Since the graft is located within the interbody space, any local deformation of the structure leads to the same amount of deformation of the graft at the interface of this local structure. As a consequence, the whole bone graft is subjected to strain. One can therefore assume that the strain of the graft at the interface with the spinal implant is directly related to the strain of the spinal implant.

In a linear elastic approximation, the spinal implant structure follows Hooke's law, defined as below:

$$\sigma_i = C_{ij}\varepsilon_j \quad , \qquad\qquad (\text{Eq. 1})$$

where $\sigma_i$ is the stress tensor, $C_{ij}$ is the stiffness tensor and $\varepsilon_j$ is the strain tensor.

By defining the 21 different components of the stiffness tensor $C_{ij}$ of the spinal implant at each locus of the structure, the strain experienced by the graft at the interface with the spinal implant can be controlled and limited within a desired range.

The profile of each coefficient of the strain tensor are defined along the x-, y- and z-axes in order to control the strain. In some embodiment, the stiffness tensor is defined to limit the stress in order to remain under high strain deleterious for the bone formation. In some embodiments, the strain tensor is defined to allow different targeted strain of the graft along x, y and z axis. Especially the strain along the spine main axis, called $\varepsilon_{zz}$, should be greater than in the other direction. In some embodiment, the stiffness tensor is defined to avoid shear stress experienced by the graft.

In some embodiments, the stiffness tensor of the spinal implant is defined such that the graft strain is maintained within a constant range all along the bone migration process.

The definition of the structure can made either with a direct patterning (repetition of a unit cell within a volume), a conformal patterning (optimal mesh of a volume) or a topology optimization patterning.

Referring now generally to FIGS. 1-3C and 19A-22, dynamic interbody fusion devices 10 consistent with the present disclosure comprise an interbody cage portion 12, and optionally an anterior plate 14.

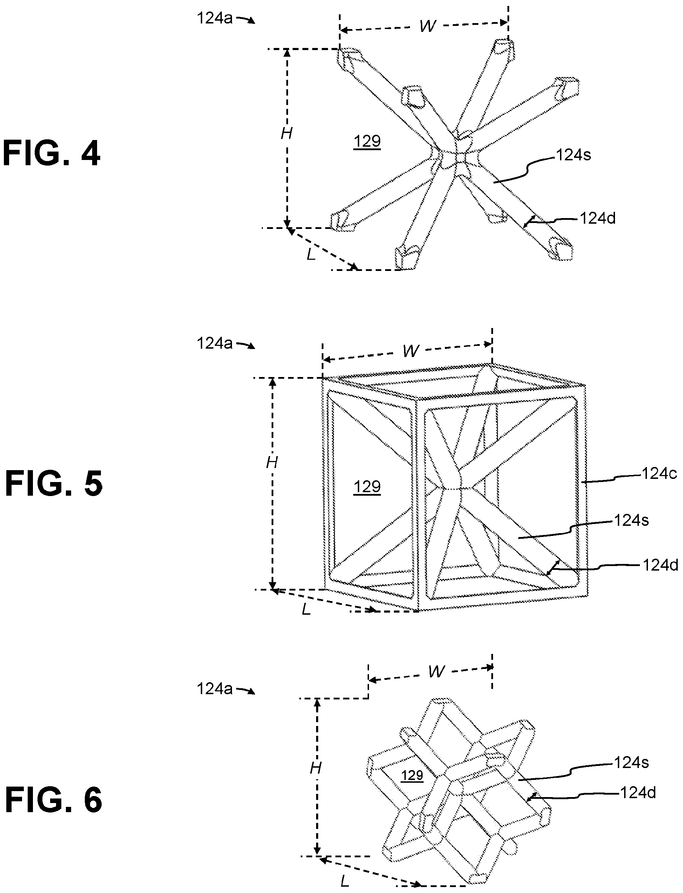

The interbody cage portion 12 is sized and shaped to be inserted between two adjacent vertebrae V1,V2, for example to replace an intervertebral disc that has been injured. The interbody cage portion 12 includes an inner structure 124 disposed between a superior endplate 122a and an inferior endplate 122b. The inner structure 124 includes voids to support bone growth media and/or bone graft material (not shown). The voids may be disposed between a series of struts 124s (FIGS. 4-9).

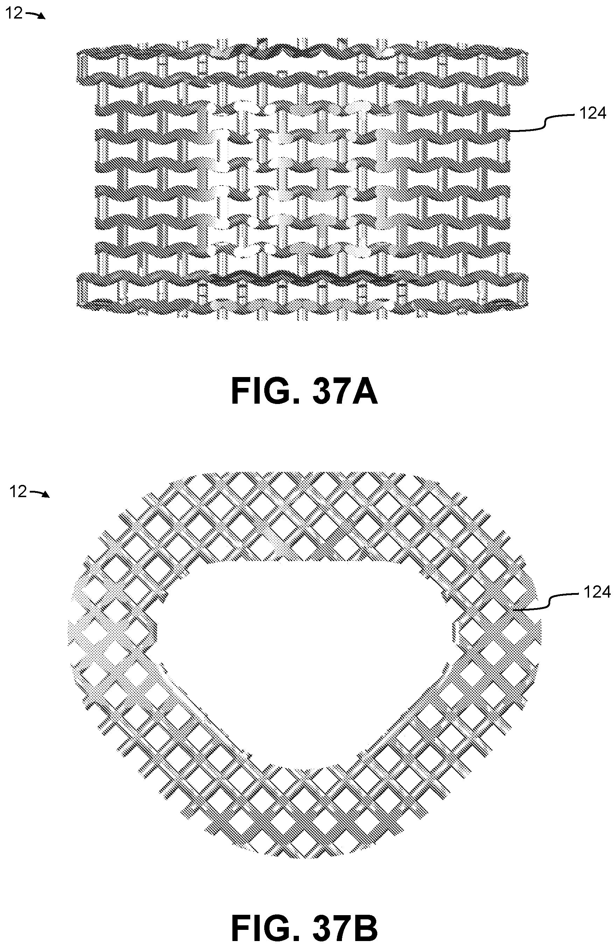

In some embodiments, such as those consistent with the embodiment specifically shown in FIGS. 37A-37B, the interbody cage consists essentially of an inner structure 124 without additional structure provided by a superior endplate 122a, an inferior endplate 122b, or an outer wall 125. In such embodiments, and after implantation into an intervertebral space of a subject, the inferior portion of the inner structure 124 will be in direct contact with the inferior adjacent vertebra V2, while the superior portion of the inner structure 124 will be in direct contact with the superior adjacent vertebra V1.

In some embodiments, the superior endplate 122a includes one or more holes 126a through which bone may grow, resulting in fusion of the interbody cage 12 with the adjacent superior vertebrae V1.

In some embodiments, the inferior endplate 122b includes one or more holes (not shown) through which bone may grow, resulting in fusion of the interbody cage 12 with the adjacent inferior vertebrae V2.

The anterior plate 14 is configured to be anchored to the adjacent superior vertebrae V1 (e.g., by driving an anchor (e.g., an anchor nail or a screw) through one or more anchoring holes 146a of a superior plate portion 142a into the adjacent superior vertebrae V1), the adjacent inferior vertebrae V2 (e.g., by driving an anchor through one or more anchoring holes 146b of an inferior plate portion 142b into the adjacent inferior vertebrae V2), and the interbody cage portion 12 (e.g., by driving an anchor through one or more anchoring holes 146c of a central plate portion 142c into the interbody cage portion 12). In some embodiments, a central plate portion 142c is disposed between the superior inner structure 144a and the inferior inner structure 144b. In some embodiments, the anterior plate 14 includes a superior inner structure 144a that includes voids and/or an inferior inner structure 144b that includes voids that, in combination with the designs of the superior plate portion 142a, the inferior plate portion 142b, and the central plate portion 142c, operate to enable a predetermine amplitude of strain at desirable vibrational frequency(ies).

In some embodiments, the interbody cage portion 12 includes an inner wall 127 defining a central void 127a. The central void 127a does not include material that forms the inner structure 124. In some embodiments, the inner wall 127 includes one or more holes 128.

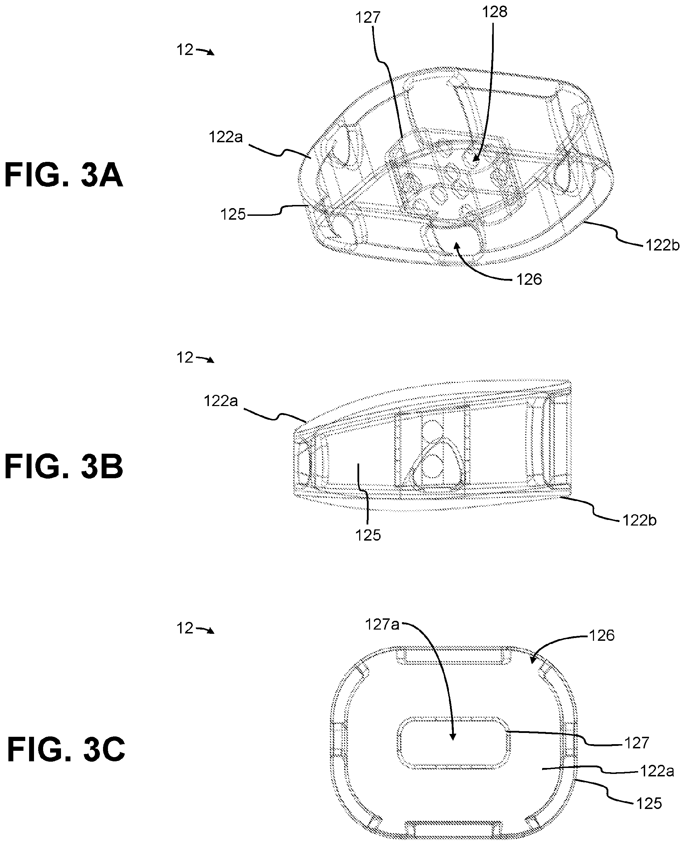

In some embodiments, such as those shown specifically in FIG. 3C, the interbody cage portion 12 includes an outer wall 125 disposed between the superior endplate 12a and the inferior endplate 122b. In some embodiments, the outer wall 125 includes one or more holes 126.

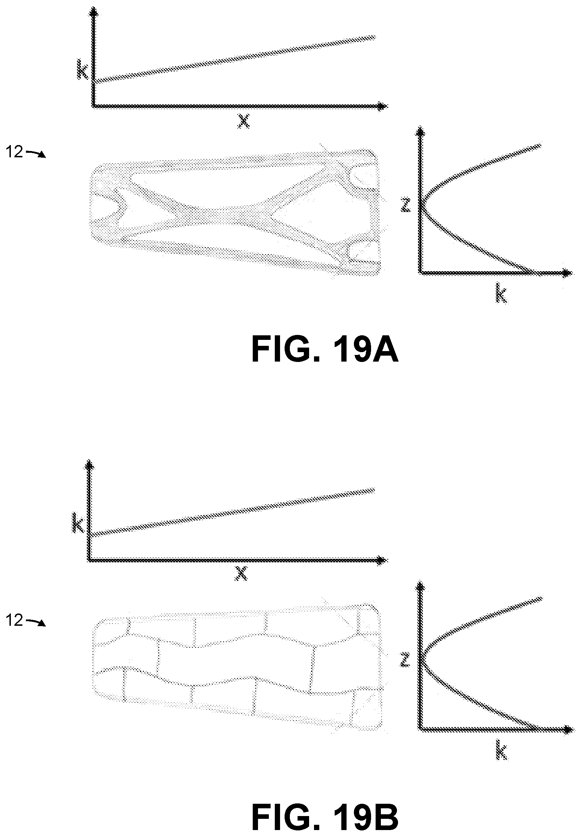

Referring now to FIG. 19A, the interbody cage portion 12 may have a controlled rigidity structure that imparts a first spring constant $k$ across the x-axis of the interbody cage portion 12, a second spring constant $k'$ across the z-axis of the interbody cage portion 12, and a third spring constant $k_y$ across the y-axis of the interbody cage portion 12. The first spring constant $k$ is related to the stiffness tensor $C_{ij}$ of Equation 1.

Referring now to FIG. 19B, the interbody cage portion 12 may have a controlled low-rigidity auxetic structure (e.g., in the inner structure 124) that imparts a first spring constant $k$ (in this example, a substantially linear spring constant $k$) across the x-axis of the interbody cage portion 12, and a second spring constant $k'$ (in this case, a nonlinear or parabolic spring constant $k'$ across the z-axis of the interbody cage portion 12. In this example, the third spring constant $k_y$ across the y-axis of the interbody cage portion 12 is not specifically graphed.

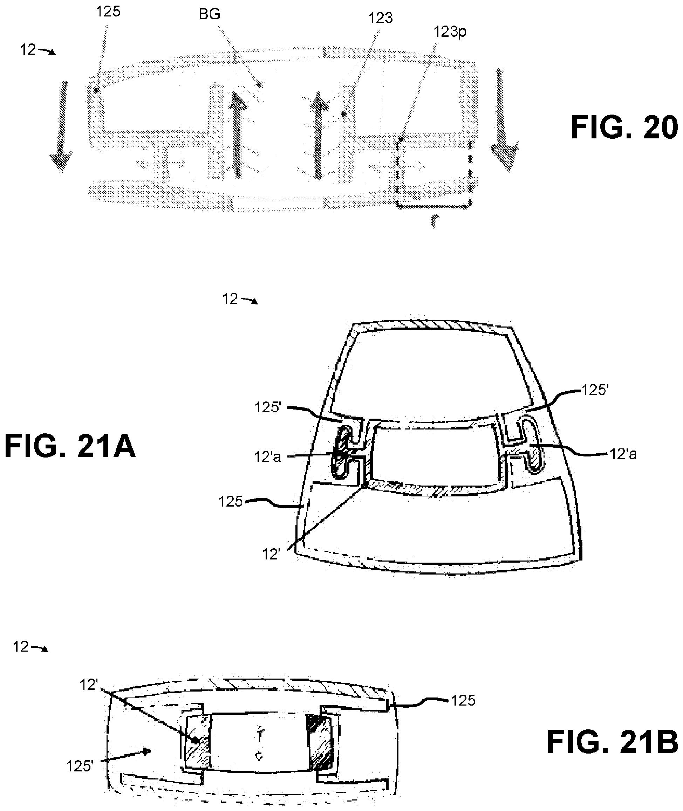

Referring now to FIG. 20, the interbody cage portion 12 may include an internal graft shield 123 configured to contact (e.g. engage or contain) bone graft material BG. A flexible pivot 123p is disposed between the internal graft shield 123 and the outer wall 125, and enables the internal graft shield 123 to move in a direction contrary to movement of the outer wall 125 (see arrows). The flexible pivot 123p defines a radius $r$ that can be chosen to provide a predetermined amplitude ratio between the flexible pivot 123p and the outer wall 125. Without wishing to be bound by theory, it is believed that the contrary movement of the internal graft shield 123 relative to the outer wall 125 enabled by the flexible pivot 123p reduces displacements of the bone graft material BG, or portions thereof, in response to compressive forces or vibrations applied to the outer wall 125 of the interbody cage portion 12. The predetermined amplitude ratio in some embodiments is defined as shown in Equation 2 below:

$$Inner\ Displacement = -\left(\frac{Distance\ from\ the\ pivot\ \mathbf{123p}\ to\ the\ outer\ wall\ \mathbf{125}}{Distance\ from\ the\ pivot\ \mathbf{123p}\ to\ the\ inner\ wall\ \mathbf{127}}\right) \times Outer\ Displacement$$

(Eq. 2)

Turning now to FIGS. 21A-21B, the interbody cage portion 12 may include a pair of opposing internal guide rails 125', for example disposed continuously/contiguously with the side walls 125, and configured to receive a pair of graft shield rails 12'a that are associated with graft shield 12'. The graft shield 12' is not attached to the interbody cage portion 12 in these embodiments; rather, the graft shield 12' is free to move within the interbody cage portion 12, with the largest freedom of movement being along the z-axis (e.g., vertically).

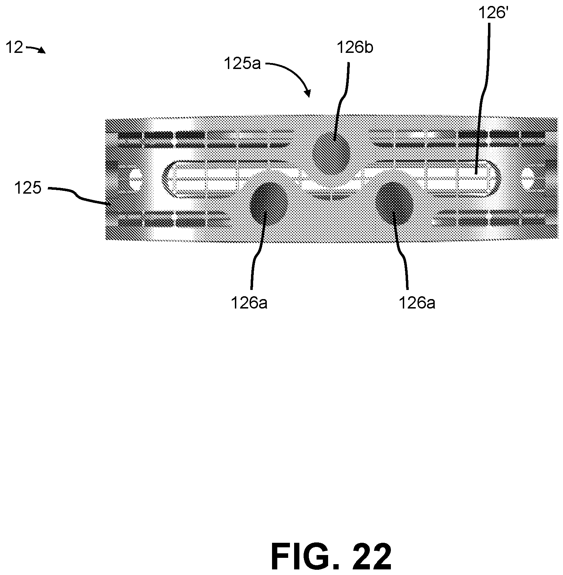

As shown in FIG. 22, interbody cage portions 12 consistent with some embodiments include a slit 126', such as a lateral slit 126', disposed in the side wall 125. In some embodiments, the slit 126' is disposed at least partially between one or more caudal fixation holes 126a and one or more cranial fixation holes 126b. In some embodiments, for example those in which the interbody cage portion 12 is intended to be implanted into a subject by an anterior lumbar interbody fusion ("ALIF") procedure, the lateral slit 126' may be disposed in an anterior portion of the side wall 125.

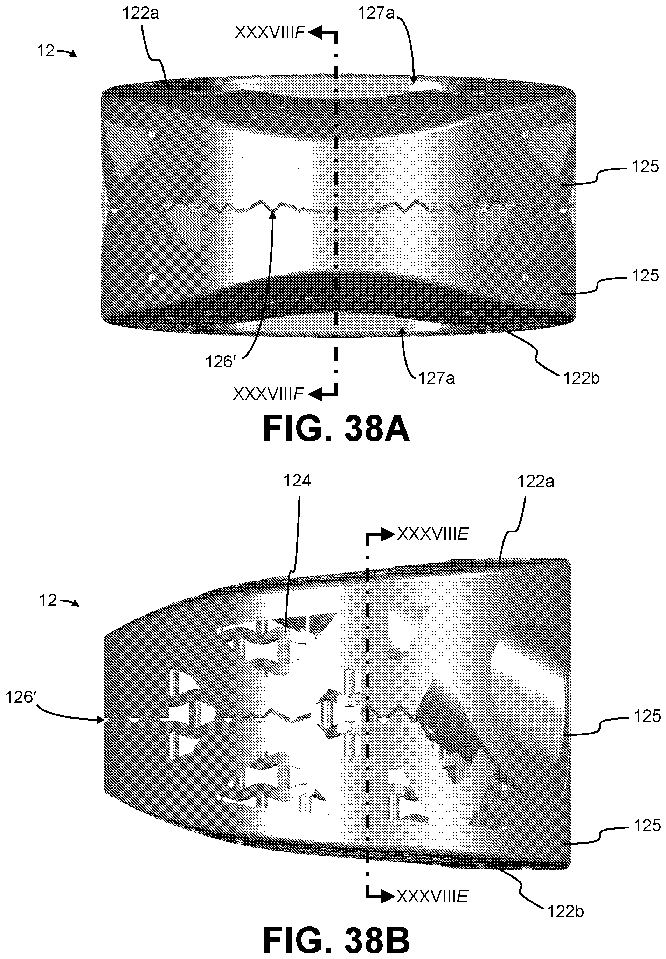

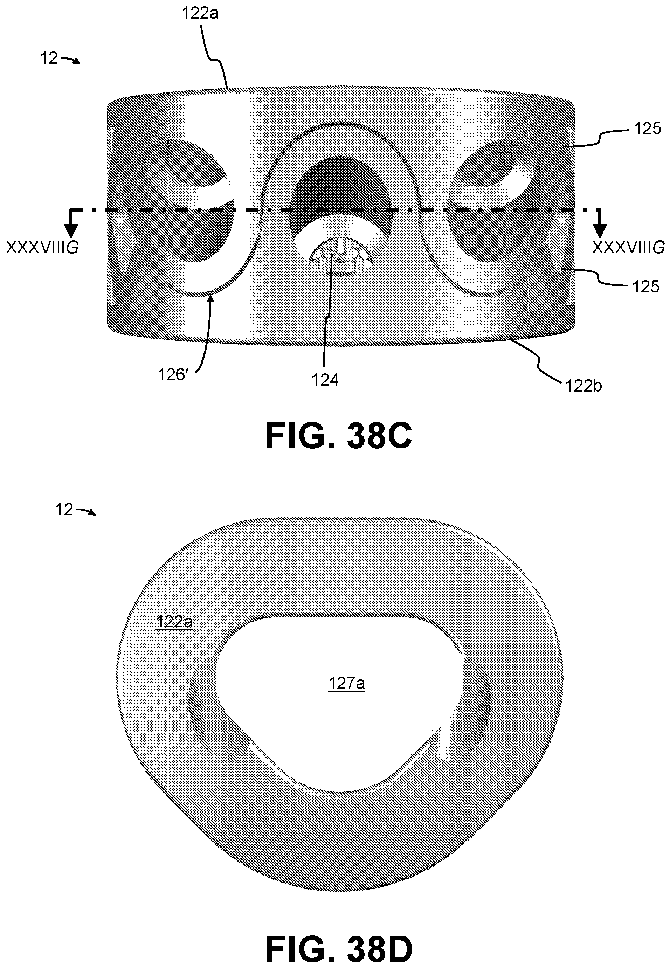

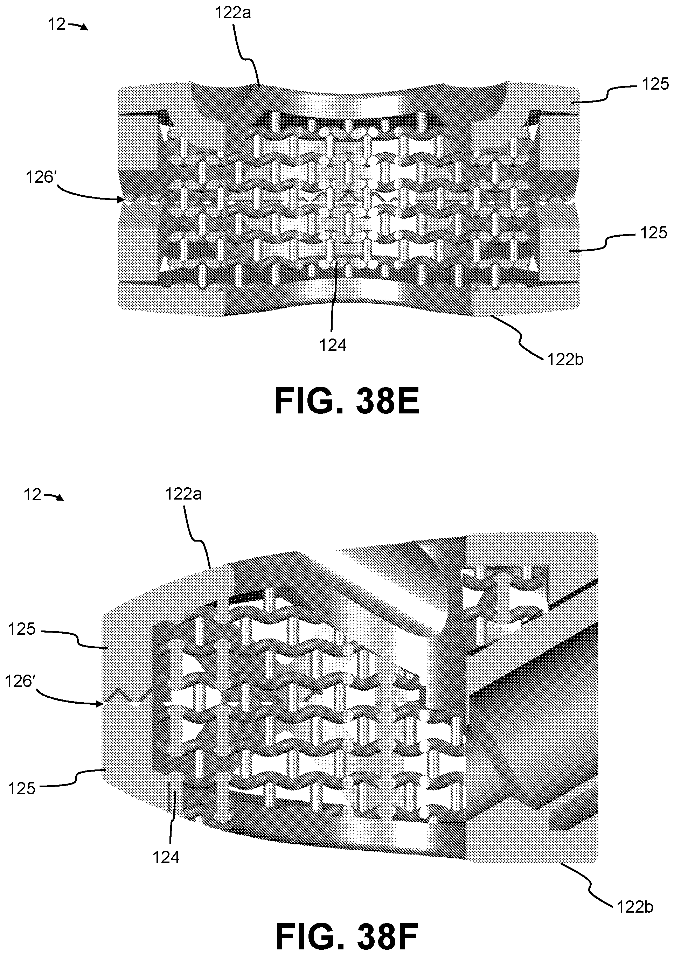

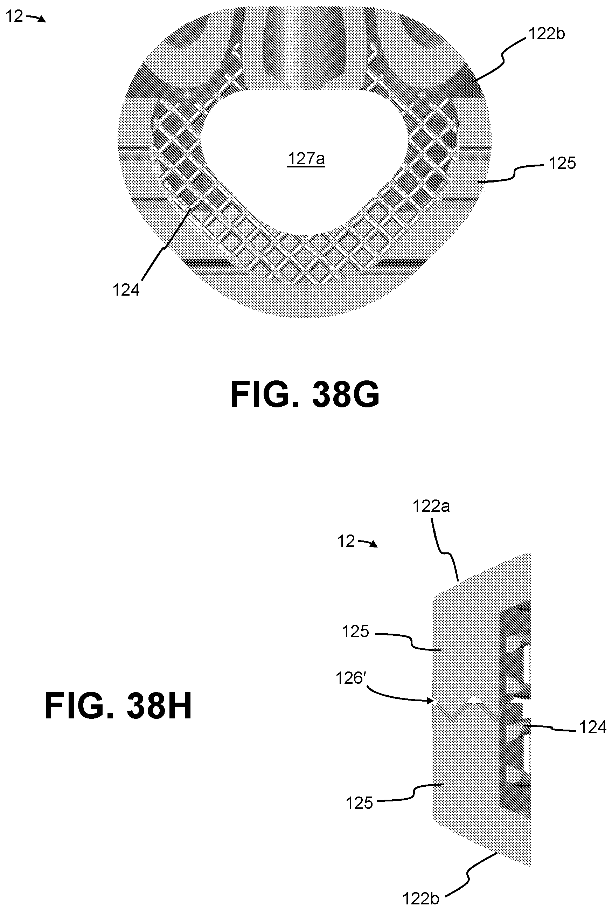

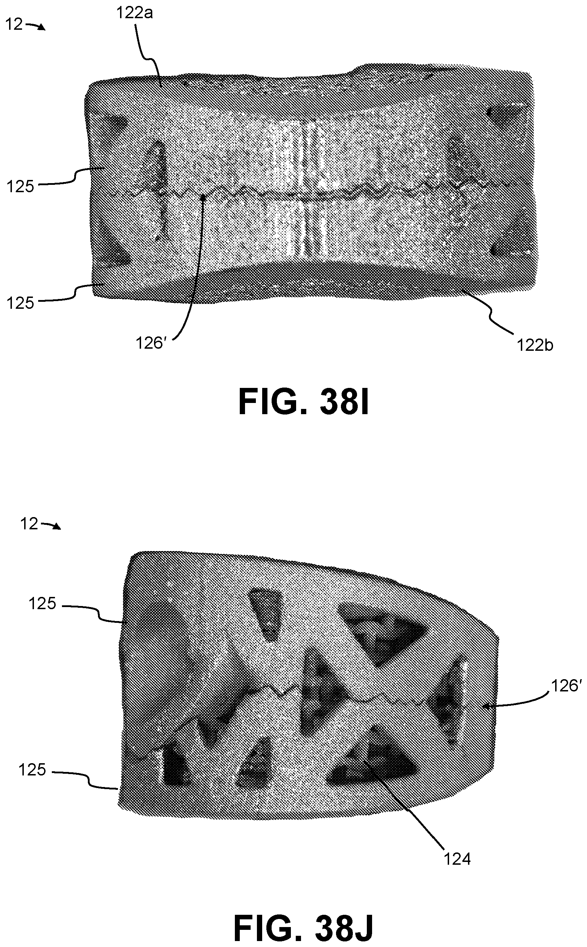

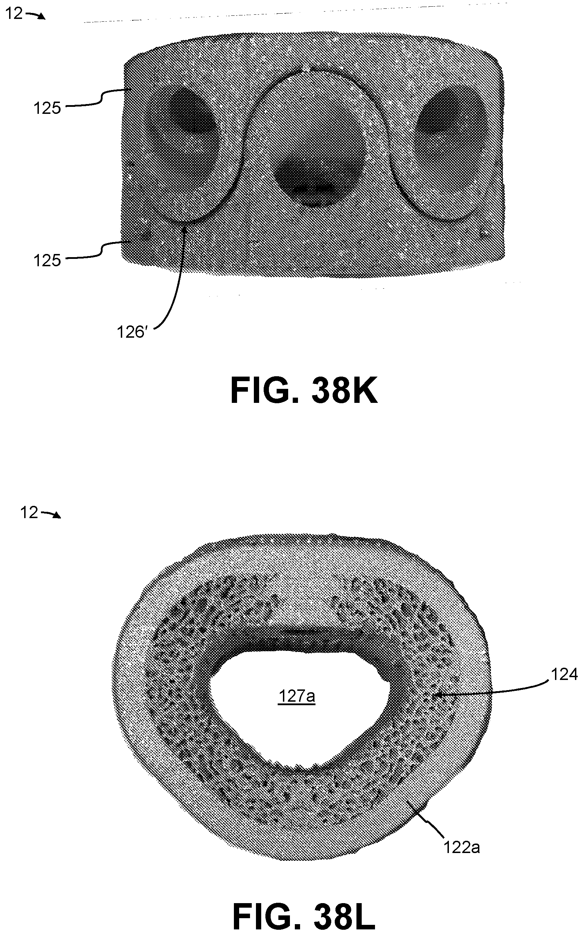

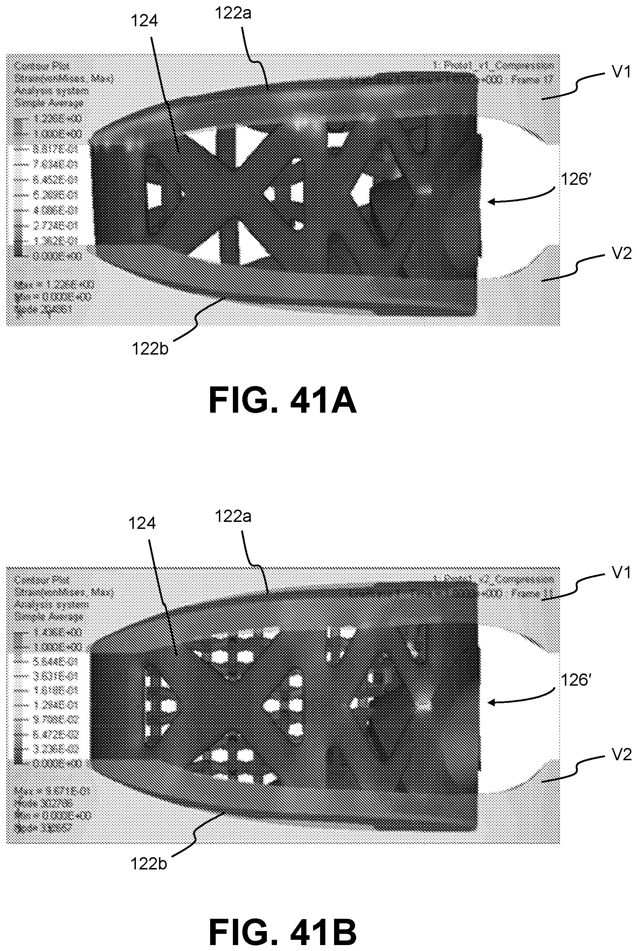

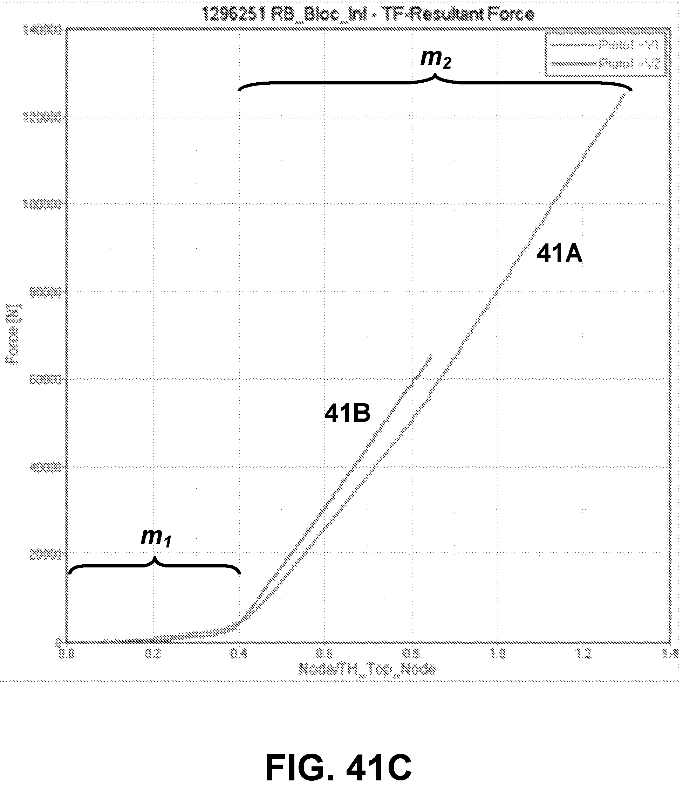

In some embodiments, such as those consistent with the embodiment specifically shown in FIGS. 38A-38H, the slit 126' is located all around or substantially around the interbody cage portion 12 of the spinal implant device 10. In such embodiments, the lower structure (e.g., the inferior endplate 122b) and the upper structure (e.g., the superior endplate 122a) are associated only (or substantially only) through their associations with the inner structure 124 (in this depicted example, the auxetic lattice). The slit 126' in the outer wall 125 in such embodiments may act as a mechanical stop to prevent compression along the z-axis more than a predetermined amount; when the slit 126' collapses under compression load, the interaction of the complementary pattern (e.g., teeth) located on the interface between the two ends of the side wall 125 prevent shear and bending motion of the implant 10. In these embodiments, the stiffness of the inner structure 124 along the z-axis is relatively low. Under compression, this embodiment of the spinal implant 10 has a double slope response to compressive force along the z-axis in terms of stiffness shown in FIG. 41A-41C. In the beginning of the deformation, only the inner structure 124 is compressed. Once the mechanical stop is reached (e.g., when the slit 126' collapses), both the inner structure 124 and the outer wall 125 are compressed, resulting in a much greater stiffness or resistance to the compressive force along the z-axis.. The slopes (e.g., resistance to compression) can be adjusted (e.g.,

tuned) either by adjusting the parameters of the inner structure 124 (e.g., strut diameter 124d,124d', length, initial deflection 124f' between adjacent flexion struts 124f, etc.) and/or the thickness and design of the outer wall 125.

Referring now specifically to FIGS. 41A-41C, two *in silico* model dynamic interbody fusion devices 12 including a slit 126' completely separating the superior endplate 122a and the inferior endplate 122b and including different inner structures 124 were prepared *in silico* as shown in FIGS. 41A-41B. In these embodiments, the inner structure 124 primarily absorbs mechanical load imparted on the dynamic interbody fusion device 12 while the slit 126' remains in an "open" configuration. When the slit126' collapses, or adopts a "closed" configuration, the superior endplate 122a, the inferior endplate 122b, and the side wall 125 (when present) then primarily absorb mechanical load imparted on the device 12. For example, when compressed along the z-axis, the devices 12 shown in FIGS. 41A-41B initially resist the compression force at a first resistance slope $m_1$ that is related to the specific dimensions and parameters of the inner structure 124 of that device 12. Once the slit 126' collapses under the compressive force, however, the device 12 resists further compression at a second resistance slope $m_2$ that is related to the specific dimensions and parameters of the superior endplate 122a, the inferior endplate 122b, and the side wall 125 (when present). Thus, the device 12 may be tuned to resist imparted mechanical load along any axis by a desired amount by selecting the dimensions, materials, parameters, and orientation of the inner structure 124, the superior endplate 122a, the inferior endplate 122b, and the side wall 125.

The caudal fixation holes 126a and/or cranial fixation holes 126b may, in some embodiments, also define an instrument interface area 125a to which the interbody cage portion 12 may be affixed (e.g., temporarily affixed) to an implantation instrument.

## 2. Repeatable Lattice Units

Referring now generally to FIGS. 4-9 and 23A-25B, the inner structure 124 includes a lattice pattern 124a formed by struts 124s, and/or flexion struts 124f, and/or cage struts 124c; and voids 129 disposed between the structs 124s, 124f, 124c. The struts 124s and flexion struts 124f may have any suitable cross-sectional shape, such as a square, a circle, a rhombus, a rectangle, a trapezoid, an oval, a triangle, a quadrilateral, a pentagon, a hexagon, etc. In some embodiments, the struts 124s have a circular cross-sectional shape with a strut diameter 124d. In some embodiments, the flexion struts 124f have a circular cross-sectional shape with a flexion strut diameter 124fd.

Generally, the resonance frequency(ies) of the inner structure 124 can be selected by using one or more lattice patterns 124a; using struts 124s, flexion struts 124f, and/or cage struts 124c of differing dimensions and diameters; and the material(s) from which the struts 124s, flexion struts 124f, and cage struts 124c are constructed.

As shown in FIG. 4, the lattice pattern 124a in some embodiments is an orthorhombic body-centered lattice pattern including eight struts 124s. In some embodiments, each strut 124s has a similar length and diameter 124d. In some embodiments, each strut 124s has an identical length and diameter 124d. The orthorhombic body-centered lattice pattern has a repeatable unit with a width W, a length L, and a height H. In some embodiments, the width W is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the length L is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the height H is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the width W, length L, and height H are substantially similar. In some embodiments, the width W, length L, and height H are identical.

Referring now to FIG. 5, the lattice pattern 124a in some embodiments is a caged orthorhombic body-centered lattice pattern including eight struts 124s surrounded by cage struts 124c. In some embodiments, each strut 124s has a similar length and diameter 124d. In some embodiments, each strut 124s has an identical length and diameter 124d. The caged orthorhombic body-centered lattice pattern has a repeatable unit with a width W, a length L, and a height H. In some embodiments, the width W is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the length L is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the height H is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the width W, length L, and height H are substantially similar. In some embodiments, the width W, length L, and height H are identical.

As shown in FIG. 6, the lattice pattern 124a in some embodiments is a dode-thin lattice pattern including 16 struts 124s. In some embodiments, each strut 124s has a similar length and diameter 124d. In some embodiments, each strut 124s has an identical length and diameter 124d. The dode-thin lattice pattern has a repeatable unit with a width W, a length L, and a height H. In some embodiments, the width W is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the length L is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the height H is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the width W, length L, and height H are substantially similar. In some embodiments, the width W, length L, and height H are identical.

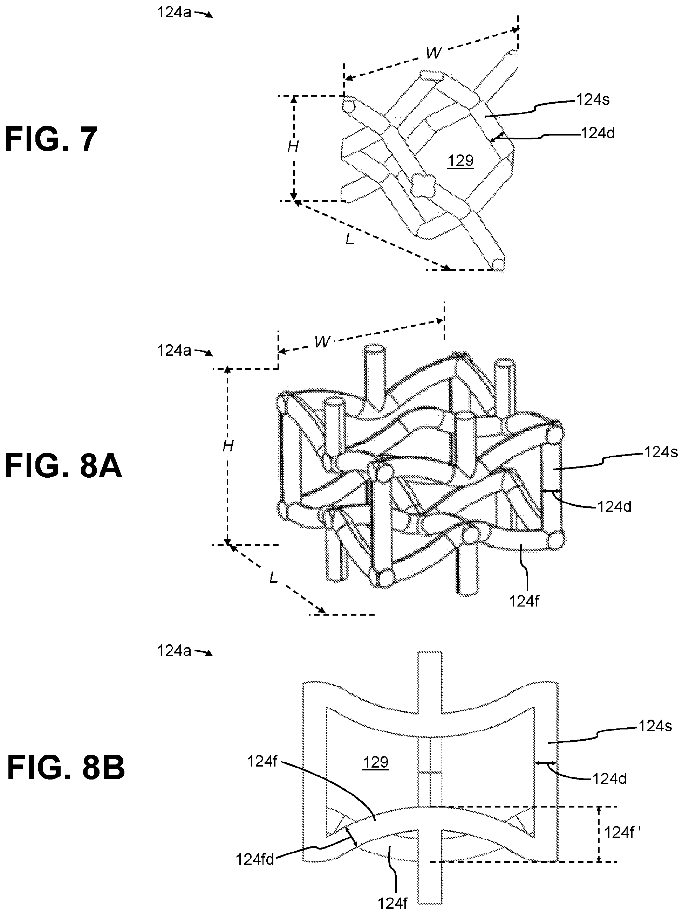

Referring now to FIG. 7, the lattice pattern 124a in some embodiments is a diamond lattice pattern including 16 struts 124s. In some embodiments, each strut 124s has a similar length and diameter 124d. In some embodiments, each strut 124s has an identical length and diameter 124d. The diamond lattice pattern has a repeatable unit with a width W, a length L, and a height H. In some embodiments, the width W is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the length L is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the height H is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the width W, length L, and height H are substantially similar. In some embodiments, the width W, length L, and height H are identical.

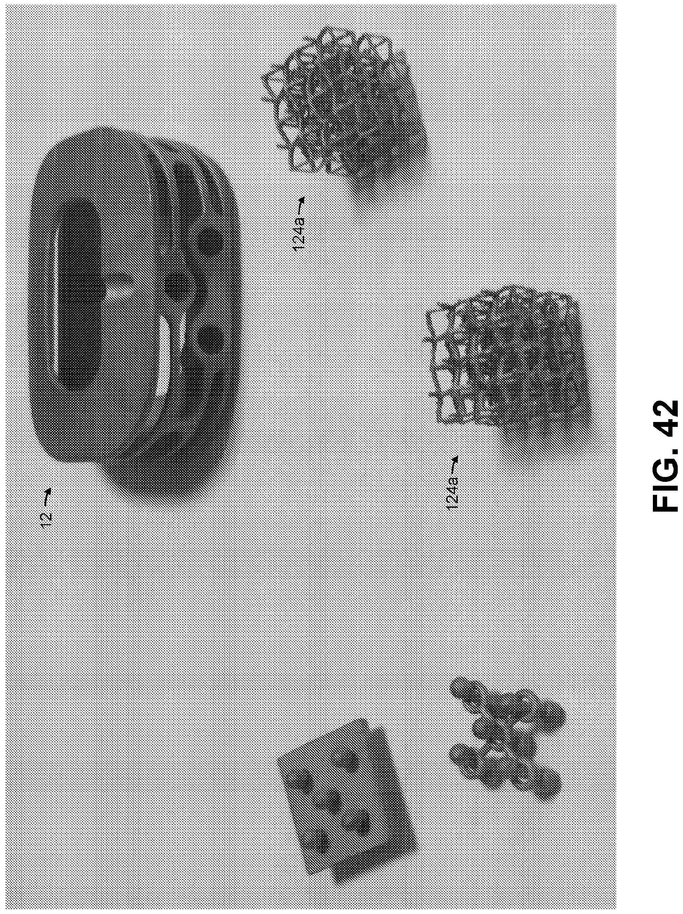

As shown in FIGS. 8A-8B and 42, the lattice pattern 124a in some embodiments is an auxetic lattice pattern including struts 124s and flexion struts 124f. In some embodiments, each strut 124s has a similar length and diameter 124d. In some embodiments, each strut 124s has an identical length and diameter 124d. In some embodiments, each flexion strut 124f has a similar diameter 124fd. In some embodiments, each flexion strut 124f has an identical diameter 124fd. The auxetic pattern may feature an initial deflection 124f' between adjacent flexion struts 124f; the resonant properties of the inner structure 124 may be tuned in part by including an auxetic lattice pattern with a specific initial deflection 124f'. Generally, the deflection of the flexion struts may be defined by the maximum change in the distance 124f' between the respective apexes of two adjacent flexion struts 124f. In some embodiments, the deflection is not more than about half the height H less the diameter 124fd of the flexion struts 124f (i.e., H/2 - 124fd). In some embodiments, the deflection is not more than about 10 mm, for example not more than about 10 mm, not more than about 9.9 mm, not more than about 9.8 mm, not more than about 9.7 mm, not more than about 9.6 mm, not more than about 9.5 mm, not more than about 9.4 mm, not more than about 9.3 mm, not more than about 9.2 mm, not more than about 9.1 mm, not more than about 9 mm, not more than about 8.9 mm, not more than about 8.8 mm, not more than about 8.7 mm, not more than about 8.6 mm, not more than about 8.5 mm, not more than about 8.4 mm, not more than about 8.3 mm, not more than about 8.2 mm, not more than about 8.1 mm, not more than about 8 mm, not more than about 7.9 mm, not more than about 7.8 mm, not more than about 7.7 mm, not more than about 7.6 mm, not more than about 7.5 mm, not more than about 7.4 mm, not more than about 7.3 mm, not more than about 7.2 mm, not more than about 7.1 mm, not more than about 7 mm, not more than about 6.9 mm, not more than about 6.8 mm, not more than about 6.7 mm, not more than about 6.6 mm, not more than about 6.5 mm, not more than about 6.4 mm, not more than about 6.3 mm, not more than about 6.2 mm, not more than about 6.1 mm, not more than about 6 mm, not more than about 5.9 mm, not more than about 5.8 mm, not more than about 5.7 mm, not more than about 5.6 mm, not more than about 5.5 mm, not more than about 5.4 mm, not more than about 5.3 mm, not more than about 5.2 mm, not more than about 5.1 mm, not more than about 5 mm, not more than about 4.9 mm, not more than about 4.8 mm, not more than about 4.7 mm, not more than about 4.6 mm, not more than about 4.5 mm, not more than about 4.4 mm, not more than about 4.3 mm, not more than about 4.2 mm, not more than about 4.1 mm, not more than about 4 mm, not more than about 3.9 mm, not more than about 3.8 mm, not more than about 3.7 mm, not more than about 3.6 mm, not more than about 3.5 mm, not more than about 3.4 mm, not more than about 3.3 mm, not more than about 3.2 mm, not more than about 3.1 mm, not more than about 3 mm, not more than about 2.9 mm, not more than about 2.8 mm, not more than about 2.7 mm, not more than about 2.6 mm, not more than about 2.5 mm, not more than about 2.4 mm, not more than about 2.3 mm, not more than about 2.2 mm, not more than about 2.1 mm, not more than about 2 mm, not more than about 1.9 mm, not more than about 1.8 mm, not more than about 1.7 mm, not more than about 1.6 mm, not more than about 1.5 mm, not more than about 1.4 mm, not more than about 1.3 mm, not more than about 1.2 mm, not more than about 1.1 mm, not more than about 1 mm, not more than about 0.9 mm, not more than about 0.8 mm, not more than about 0.7 mm, not more than about 0.6 mm, not more than about 0.5 mm, not more than about 0.4 mm, not more than about 0.3 mm, not more than about 0.2 mm, or not more than about 0.1 mm. The auxetic lattice pattern has a repeatable unit with a width W, a length L, and a height H. In some embodiments, the width W is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the length L is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the height H is about 0.1 mm to about 20 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, about 5 mm, about 5.1 mm, about 5.2 mm, about 5.3 mm, about 5.4 mm, about 5.5 mm, about 5.6 mm, about 5.7 mm, about 5.8 mm, about 5.9 mm, about 6 mm, about 6.1 mm, about 6.2 mm, about 6.3 mm, about 6.4 mm, about 6.5 mm, about 6.6 mm, about 6.7 mm, about 6.8 mm, about 6.9 mm, about 7 mm, about 7.1 mm, about 7.2 mm, about 7.3 mm, about 7.4 mm, about 7.5 mm, about 7.6 mm, about 7.7 mm, about 7.8 mm, about 7.9 mm, about 8 mm, about 8.1 mm, about 8.2 mm, about 8.3 mm, about 8.4 mm, about 8.5 mm, about 8.6 mm, about 8.7 mm, about 8.8 mm, about 8.9 mm, about 9 mm, about 9.1 mm, about 9.2 mm, about 9.3 mm, about 9.4 mm, about 9.5 mm, about 9.6 mm, about 9.7 mm, about 9.8 mm, about 9.9 mm, about 10 mm, about 10.1 mm, about 10.2 mm, about 10.3 mm, about 10.4 mm, about 10.5 mm, about 10.6 mm, about 10.7 mm, about 10.8 mm, about 10.9 mm, about 11 mm, about 11.1 mm, about 11.2 mm, about 11.3 mm, about 11.4 mm, about 11.5 mm, about 11.6 mm, about 11.7 mm, about 11.8 mm, about 11.9 mm, about 12 mm, about 12.1 mm, about 12.2 mm, about 12.3 mm, about 12.4 mm, about 12.5 mm, about 12.6 mm, about 12.7 mm, about 12.8 mm, about 12.9 mm, about 13 mm, about 13.1 mm, about 13.2 mm, about 13.3 mm, about 13.4 mm, about 13.5 mm, about 13.6 mm, about 13.7 mm, about 13.8 mm, about 13.9 mm, about 14 mm, about 14.1 mm, about 14.2 mm, about 14.3 mm, about 14.4 mm, about 14.5 mm, about 14.6 mm, about 14.7 mm, about 14.8 mm, about 14.9 mm, about 15 mm, about 15.1 mm, about 15.2 mm, about 15.3 mm, about 15.4 mm, about 15.5 mm, about 15.6 mm, about 15.7 mm, about 15.8 mm, about 15.9 mm, about 16 mm, about 16.1 mm, about 16.2 mm, about 16.3 mm, about 16.4 mm, about 16.5 mm, about 16.6 mm, about 16.7 mm, about 16.8 mm, about 16.9 mm, about 17 mm, about 17.1 mm, about 17.2 mm, about 17.3 mm, about 17.4 mm, about 17.5 mm, about 17.6 mm, about 17.7 mm, about 17.8 mm, about 17.9 mm, about 18 mm, about 18.1 mm, about 18.2 mm, about 18.3 mm, about 18.4 mm, about 18.5 mm, about 18.6 mm, about 18.7 mm, about 18.8 mm, about 18.9 mm, about 19 mm, about 19.1 mm, about 19.2 mm, about 19.3 mm, about 19.4 mm, about 19.5 mm, about 19.6 mm, about 19.7 mm, about 19.8 mm, about 19.9 mm, or about 20 mm. In some embodiments, the width W, length L, and height H are substantially similar. In some embodiments, the width W, length L, and height H are identical.

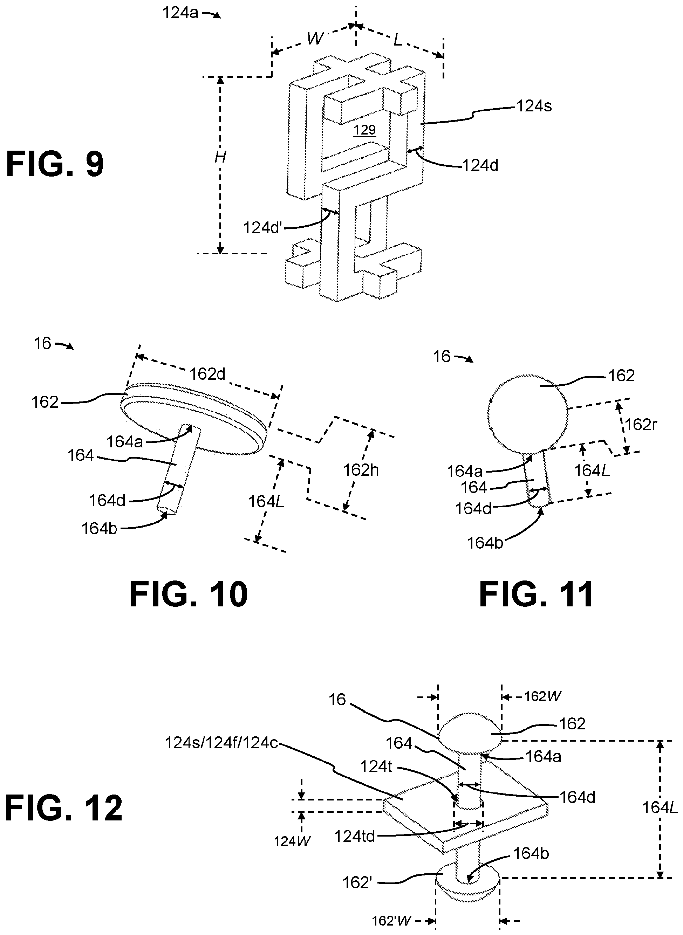

Referring now to FIG. 9, the lattice pattern 124a in some embodiments is an S-shaped lattice pattern including struts 124s. In some embodiments, the struts 124s have a circular cross-sectional shape having a diameter 124d (not shown). In other embodiments, the struts have a rectangular cross-sectional shape having a width 124d and a length 124d'. The S-shaped lattice pattern has a repeatable unit with a width W, a length L, and a height H. In some embodiments, the width W is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the length L is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the height H is about 0.1 mm to about 5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, about 2.5 mm, about 2.6 mm, about 2.7 mm, about 2.8 mm, about 2.9 mm, about 3 mm, about 3.1 mm, about 3.2 mm, about 3.3 mm, about 3.4 mm, about 3.5 mm, about 3.6 mm, about 3.7 mm, about 3.8 mm, about 3.9 mm, about 4 mm, about 4.1 mm, about 4.2 mm, about 4.3 mm, about 4.4 mm, about 4.5 mm, about 4.6 mm, about 4.7 mm, about 4.8 mm, about 4.9 mm, or about 5 mm. In some embodiments, the width W, length L, and height H are substantially similar. In some embodiments, the width W, length L, and height H are identical.

In some embodiments, the inner structure 124 includes only one lattice pattern 124a. In other embodiments, the inner structure 124 includes more than one lattice pattern 124a, such as 2 lattice patterns 124a, 3 lattice patterns 124a, 4 lattice patterns 124a, 5 lattice patterns 124a, 6 lattice patterns 124a, 7 lattice patterns 124a, 8 lattice patterns 124a, 9 lattice patterns 124a, 10 lattice patterns 124a, or more than 10 lattice patterns 124a.

Regardless of the lattice pattern(s) 124a, the inner structure 124 includes a ratio of the volume of voids 129 to the volume of struts 124s,124f,124c of about 100:1 to about 1:100, for example about 100:1, about 95:1, about 90:1, about 85:1, about 80:1, about 75:1, about 70:1, about 65:1, about 60:1, about 55:1, about 50:1, about 45:1, about 40:1, about 35:1, about 30:1, about 25:1, about 20:1, about 15:1, about 10:1, about 5:1, about 1:1, about 1:5, about 1:10, about 1:15, about 1:20, about 1:25, about 1:30, about 1:35, about 1:40, about 1:45, about 1:50, about 1:55, about 1:60, about 1:65, about 1:70, about 1:75, about 1:80, about 1:85, about 1:90, about 1:95, or about 1:100.

In some embodiments, the distribution of the lattice pattern units through the inner structure 124 is substantially consistent throughout the inner structure 124. In other embodiments, the density of lattice pattern units through the inner structure 124 is unequal, for example including one or more areas of relatively high density and one or more areas of relatively low density. In some embodiments, the density of lattice pattern units through the inner structure 124 represents a gradient, such as a linear gradient, a quadratic gradient, an exponential gradient, or a logarithmic gradient (e.g., a natural logarithmic gradient and/or a base-10 logarithmic gradient).

In some embodiments, the inner structure 124 is formed using an additive (e.g., 3D printing) process. In such embodiments, the inner structure 124 may consist or consist essentially of any material that can be used in an additive (e.g., 3D printing) process that is also biocompatible. Non-limiting examples of such materials include titanium, poly-ethyl ethyl ketone (PEEK), stainless steel (e.g., 316L or BIODUR 108 stainless steel), cobalt chrome alloy, a ceramic, or a combination thereof.

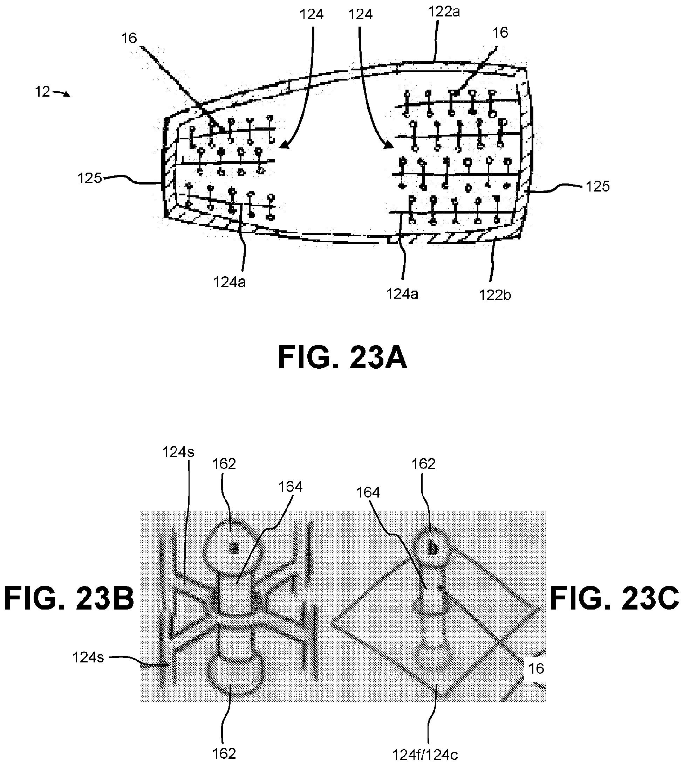

Turning now to FIGS. 23A-23C, interbody cage portions 12 consistent with some embodiments of the present disclosure include a plurality of free resonators 16 disposed on lattice structures 124a/124s and/or on transverse plates 124f/124c of the inner structure 124. In some embodiments, all of the free resonators 16 have stem portions 164 that are aligned, for example parallel to the z-axis as shown specifically in FIG. 23A.

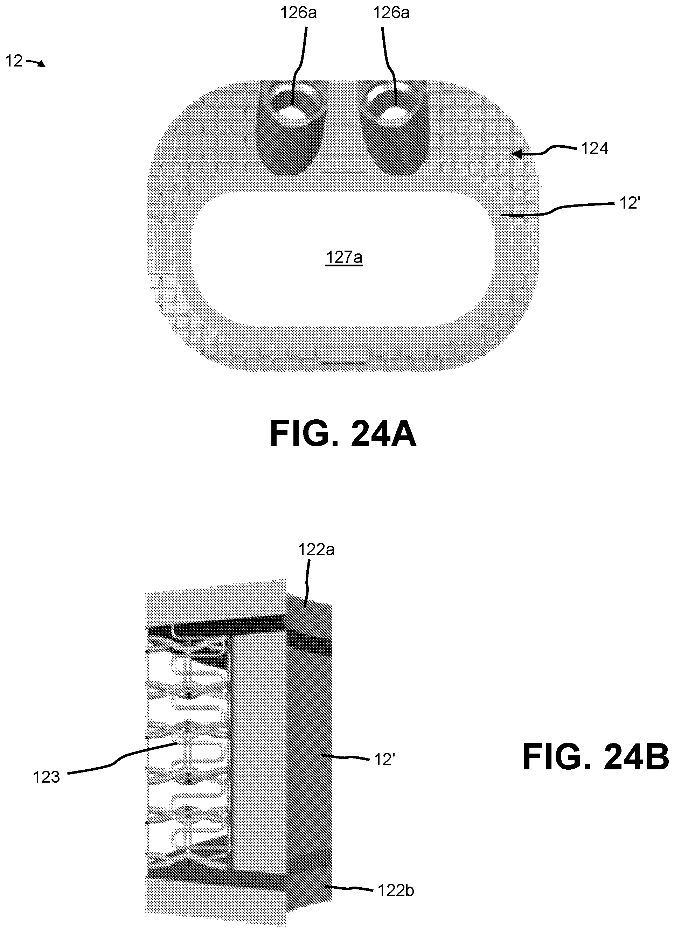

Referring now to FIGS 24A-24B, interbody cage portions 12 consistent with some embodiments include both a lattice structure in the inner structure 124 and a graft shield 12' surrounding the cavity 127a. The graft shield 12' is associated with the superior endplate 122a and/or the inferior endplate 122b via springs 123.

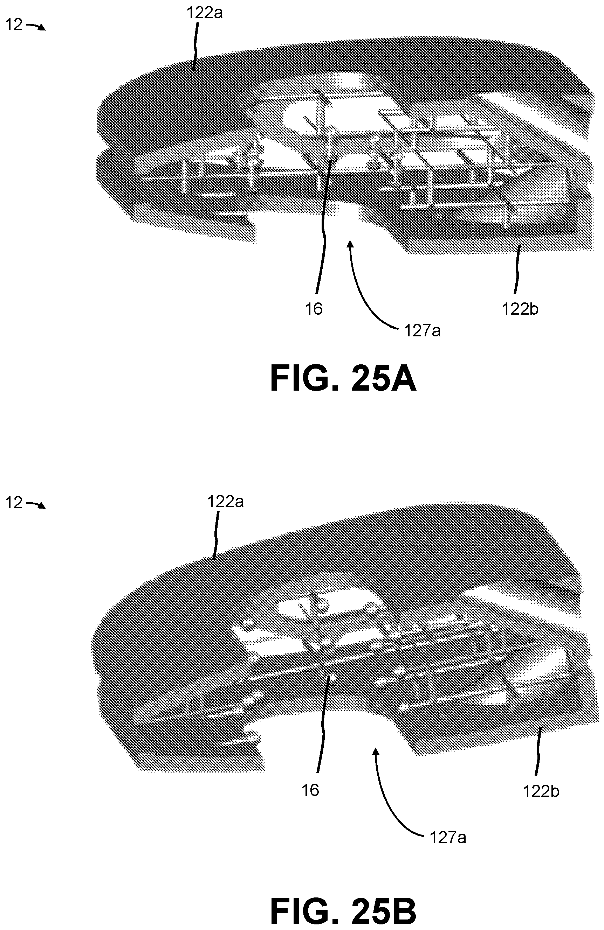

As shown in FIGS. 25A-25B, interbody cage portions 12 consistent with some embodiments include resonating elements 16 (FIG. 25A) and/or free resonators 16 (FIG. 25B) disposed within the cavity 127a.

3. Resonating Elements

Referring now generally to FIGS. 10-16, dynamic interbody fusion devices 10 consistent with the present disclosure may include one or more resonating elements 16. The resonating element(s) 16 may be sized and shaped to vibrate at a desired frequency, for example to amplify or dampen vibration of the dynamic interbody fusion device 10 at predetermined frequency(ies).

Generally, resonating elements 16 include a mass 162 associated with a stem portion 164, such as at or near one end of the stem portion 164. When the stem portion 164 (e.g., the opposite end of the stem portion 164) is affixed to or otherwise associated with the dynamic interbody fusion device 10 (e.g., with the inner structure 124), the resonating elements 16 act as harmonic resonators, vibrating in response to wave(s) applied to the dynamic interbody fusion device 10 when the wave includes a frequency in resonance with the vibrational frequency(ies) of the resonating element 16. In some embodiments, the resonating element 16 has a fundamental harmonic frequency of about 10 kHz to about 100 kHz, for example about 10 kHz, about 15 kHz, about 20 kHz, about 25 kHz, about 30 kHz, about 35 kHz, about 40 kHz, about 45 kHz, about 50 kHz, about 55 kHz, about 60 kHz, about 65 kHz, about 70 kHz, about 75 kHz, about 80 kHz, about 85 kHz, about 90 kHz, about 95 kHz, or about 100 kHz.

The resonating element 16 may be formed of any suitable biocompatible material, such as titanium, PEEK, stainless steel (e.g., 316L or BIODUR 108 stainless steel), cobalt chrome alloy, a ceramic, or a combination thereof. In some embodiments, the resonating element 16 is formed by an additive manufacturing process, such as 3D printing. In some embodiments, the resonating element 16 is formed concurrently with formation of the inner structure 124, for example by 3D printing methods.

Referring now specifically to FIG. 10, a resonating element 16 consistent with one embodiment of the present disclosure comprises a disc-shaped mass portion 162 disposed at a distal end 164a of a stem portion 164. The disc-shaped mass portion 162 has a thickness 162h and a diameter 162d. In some embodiments, the thickness 162h is about 0.1 mm to about 2.5 mm, for example about 0.1 mm, about 0.2 mm, about 0.3 mm, about 0.4 mm, about 0.5 mm, about 0.6 mm, about 0.7 mm, about 0.8 mm, about 0.9 mm, about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2 mm, about 2.1 mm, about 2.2 mm, about 2.3 mm, about 2.4 mm, or about 2.5 mm. In some embodiments, the diameter 162d is about 0.5 mm to about 10 mm, for example about 0.5 mm, about 0.6 mm, about 0.7 mm, about 0.8 mm, about 0.9 mm, about 1 mm, about 1.2 mm, about 1.4 mm, about 1.6 mm, about 1.8 mm, about 2 mm, about 2.2 mm, about 2.4 mm, about 2.6 mm, about 2.8 mm, about 3 mm, about 3.2 mm, about 3.4 mm, about 3.6 mm, about 3.8 mm, about 4 mm, about 4.2 mm, about 4.4 mm, about 4.6 mm, about 4.8 mm, about 5 mm, about 5.2 mm, about 5.4 mm, about 5.6 mm, about 5.8 mm, about 6 mm, about 6.2 mm, about 6.4 mm, about 6.6 mm, about 6.8 mm, about 7 mm, about 7.2 mm, about 7.4 mm, about 7.6 mm, about 7.8 mm, about 8 mm, about 8.2 mm, about 8.4 mm, about 8.6 mm, about 8.8 mm, about 9 mm, about 9.2 mm, about 9.4 mm, about 9.6 mm, about 9.8 mm, or about 10 mm. The stem portion 162 has a length 164L and a diameter 164d. In use, the resonating element 16 is attached to the inner structure 124 at the proximal end 164b of the stem portion 164.

As shown in FIG. 11, a resonating element 16 consistent with another embodiment of the present disclosure includes a spherical mass portion 162 disposed at a distal end 164a of a stem portion 164. The spherical mass portion 162 has a radius 162r; the stem portion 164 has a stem diameter 164d and a length 164L. In use, the resonating element 16 is attached to the inner structure 124 at the proximal end 164b of the stem portion 164

The resonant frequency(ies) of the resonating element 16 are determined (e.g., tunable) by selecting the size and shape of the mass portion 162, the length 164L of the stem portion 164, and the material from which the resonating element 16 is made. Generally, a higher mass of the mass portion 162 and longer length 164L of the stem portion 164 result in lower resonant frequencies compared to a resonating element 16 made from the same material but having a smaller mass of the mass portion 162 and shorter length 164L of the stem portion 164.

Referring now to FIGS. 11 and 42, a resonating element 16 consistent with the present disclosure may be a free-resonating element 16 that is retained by, but not affixed to, the inner structure 124. In some embodiments, the free-resonating element 16 includes a first mass portion 162 disposed at a first end 164a of a stem portion 164, and a second mass portion 162' disposed at a second end 164b of the stem portion 164. The first mass portion 162 may feature a similar or identical size and shape as the second mass portion 162' in some embodiments; in other embodiments, the first mass portion 162 has a different size and/or shape than the second mass portion 162'. The stem portion 164 extends through a hole 124t in the inner structure 124 that has a slightly larger diameter 124td than the diameter 164d of the stem portion 164. The stem portion 164 has a length 164L greater than the width 124W of the strut 124s/124f/124c through which the stem portion 164 extends. In such embodiments, the resonating element 16 is free to slide (e.g., oscillate) through the strut 124s/124f/124c a maximum distance approximately equal to the length 164L of the stem portion 164 in response to an applied wave (e.g., an applied acoustical wave).

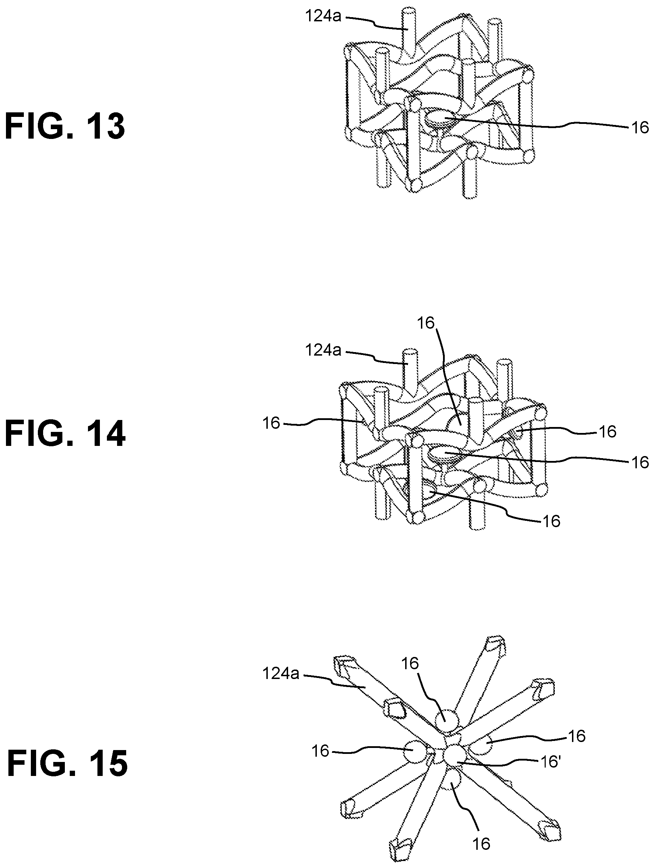

FIGS. 13-16 show representative arrangements of one or more resonating elements 16 within repeatable unit cubes of various inner structure patterns 124a. In some embodiments, such as that shown in FIG. 13, only one resonating element 16 is disposed in each repeatable unit cube of the inner structure pattern 124a. In some embodiments, the resonating element 16 is disposed in each repeatable unit cube of the inner structure 124 such that each resonating element 16 is oriented in the same direction. In other embodiments, the resonating element 16 is disposed in each repeatable unit cube of inner structure 124 such that the resonating element 16 of one repeatable unit cube may be oriented in a different direction than the resonating element 16 of an adjacent cube.

Referring now to FIG. 14, a repeatable unit cube 124a of the inner structure 124 of some embodiments includes more than one resonating element 16. In this example, the inner structure pattern 124a includes five resonating elements 16: one centrally located and four disposed in four of the eight internal corners. In this embodiment, the five resonating elements 16 are oriented in five different directions.

As shown in FIG. 15, a repeatable unit cube 124a of the inner structure 124 of some embodiments includes six resonating elements 16, each disposed at the center junction of a orthorhombic body-centered inner structure pattern 124a. Five of the six resonating elements 16 are visible in the illustrate perspective view, with the sixth resonating element 16 disposed opposite the center junction from and pointing in the opposite direction than the horizontally oriented resonating element 16'.

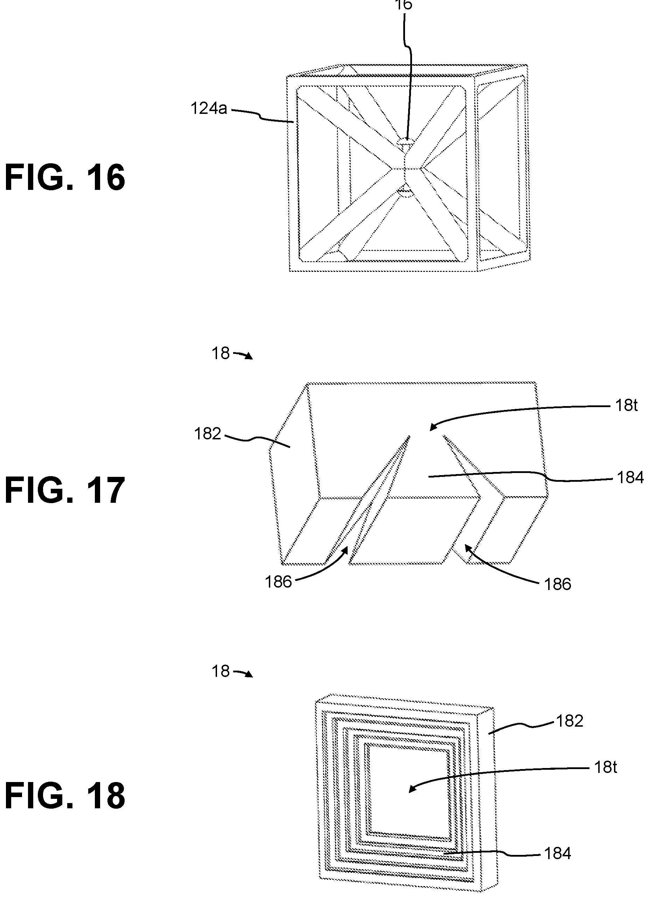

Referring now to FIG. 16, one or more free-resonating elements 16 may be incorporated into a repeatable inner structure pattern 124a in any suitable location and orientation. In the embodiment specifically shown in FIG. 16, for example, one free-resonating element 16 is disposed through a hole 124t (not shown) in the central junction of the orthorhombic body-centered inner structure pattern 124a.

4. Mechanical Load Directors

Referring now generally to FIGS. 17-18 and 39, one or more mechanical load directors 18 may be incorporated into a dynamic interbody fusion device 10 consistent with the present disclosure to redirect (e.g., focus) a propagating wave to a targeted area of interest 18t or away from a targeted area 18t. Without wishing to be bound by theory, it is believed that incorporation of one or more mechanical load directors 18 into the dynamic interbody fusion device 10 may enable redirection (e.g., focusing) of a propagating wave based at least in part on a difference in relative stiffness of the mechanical load director(s) 18 and the surrounding structural features (referred to generally herein as "mechanical structure 182").

In some embodiments, the mechanical load director 18 includes a guiding structure 184 associated with a mechanical structure 182. In some embodiments, the mechanical load director 18 further includes one or more empty spaces 186 disposed between at least a portion of the guiding structure 184 and the mechanical structure 182.

In some embodiments, such as those consistent with the embodiment specifically shown in FIG. 17, the mechanical structure 182 is a strut 124s/124f/124c, and the guiding structure 184 is defined by one or more empty spaces 186 formed in the strut 124s/124f/124c.

In some embodiments, such as those consistent with the embodiment specifically shown in FIG. 18, the mechanical structure 182 is a strut 124s/124f/124c, and the guiding structure 184 is defined by a contour of the guiding structure 184 formed in the strut 124s/124f/124c.

In some embodiments, the anterior plate 14 includes one or more mechanical load directors 18. In other embodiments, the outer wall 125 includes one or more mechanical load directors 18. In some embodiments, at least one mechanical load director 18 is disposed on the anterior plate 14 and at least one mechanical load director 18 is disposed on the interbody cage portion 12.

The mechanical load director 18 may be formed of any suitable biocompatible material, such as titanium, PEEK, stainless steel (e.g., 316L or BIODUR 108 stainless steel), cobalt chrome alloy, a ceramic, or a combination thereof. In some embodiments, the mechanical load director 18 is formed by an additive manufacturing process, such as 3D printing. In some embodiments, the mechanical load director 18 is formed concurrently with formation of the inner structure 124, for example by 3D printing methods.

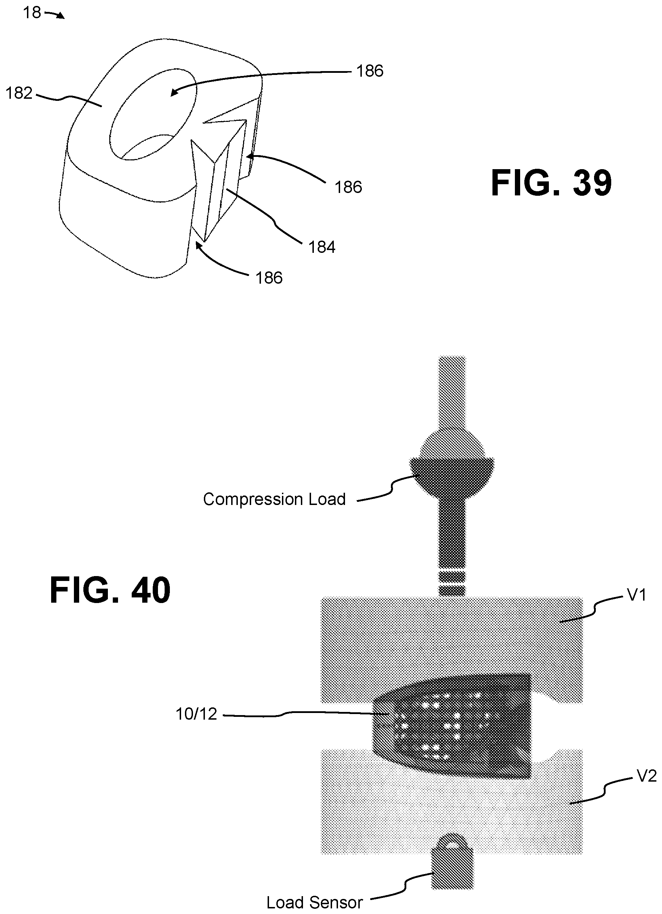

In some embodiments, such as those consistent with the embodiment specifically shown in FIG. 39, the mechanical load director 18 includes an empty space 186 disposed through the mechanical structure 182.

### 5. Methods of Treatment

Dynamic interbody fusion devices 10 may be implanted into a patient in need thereof by any suitable means, including by standard surgical techniques for implanting interbody cages into an intervertebral space. Bone graft material and/or bone growth media may be inserted into the dynamic interbody fusion device 10 if desired.

Once implanted, growth of bone tissue between the adjacent vertebrae V1,V2 may be stimulated by applying a vibrational wave to the dynamic interbody fusion device 10. The generated vibrational wave propagates through the patient's skin and tissues until it contacts the dynamic interbody fusion device 10. In some embodiments, the vibrational wave is generated by the subject's own movement. For example, vibrational wave(s) may be generated by the subject's movement while walking, running, driving, riding in a vehicle, bicycling, dancing, or the like.

The vibrational wave may then be transformed by interacting with the dynamic interbody fusion device 10. For example, one or more frequencies present in the applied wave (optionally modified after traversing through the patient's skin and tissues) may be enhanced or dampened by the resonance frequency(ies) of the inner structure 124. Application of a vibrational wave having specific frequency(ies) to the dynamic interbody fusion device 10 may therefore promote bone growth between the vertebrae V1,V2 and substantially shorten the vertebral fusion process.

### EXAMPLES

Example 1: Finite Element Analysis of Forced Excitation for the Direct Patterning Auxetic Lattice with Vertical Disconnections

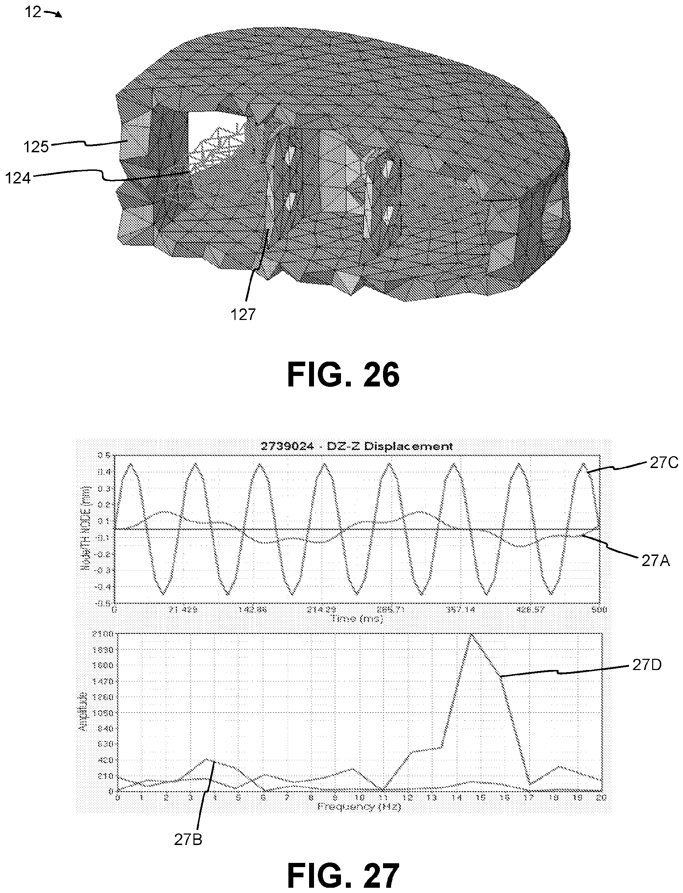

An *in silico* study of an interbody cage portion 12 consistent with FIG. 26 was performed using Altair® Radioss™ and the following parameters:

| Geometry | Lattice Unit length | 2.5 x 2.5 x 2.5 mm |
|---|---|---|
| | Lattice Patterning | Direct |
| | Lattice Strut Diameter | 0.01 mm |
| | Outer Skirt (e.g., side wall 125) Thickness | 2 mm |
| | Outer Skirt Material | Dense |
| | Upper Endplate Interface Thickness | 1 mm |
| | Lower Endplate Thickness | 1 mm |
| Material | 3D Printed Titanium Calibrated (Elastoplastic) | |
| Inner Structure 124 | Lattice Beam Elements | 1D |
| | Lattice Beam per Strut | 1 |

(continued)

| | | |
|---|---|---|
| | Lattice Nodes Tetrahedral Solid Elements | 4 |
| | Lattice Elements Target Size | 2 mm |
| Testing Conditions | Excitation Location | Base of Cage |
| | Distal Displacement | +/- 0.45 mm |
| | Resulting Distal Amplitude | 0.90 mm |
| | Forced Excitation Frequency | 15 Hz |
| Observed Output | Graft Shield Dynamic Response | FIG. 27 |

The resulting dynamic response (curves 27A and 27B) to the forced excitation (curves 27C and 27D) is shown in FIG. 27: the graft shield amplitude is 0.233 mm at a frequency of 3.8 Hz.

These results demonstrate that interbody cage portions 12 consistent with the present disclosure are capable of isolating graft material from vibrations by up to 75%.

Example 2: Modeling and Analysis of Implanted Interbody Cage Portion

To understand how interbody cage portions 12 consistent with the present disclosure may behave *in situ,* an *in silico* analysis of the interbody cage portion 12 shown in FIG. 26 (Example 1) was performed using Altair® Radioss™ and the following parameters:

| | | |
|---|---|---|
| Implant Geometry | Lattice Unit length | 2.5 x 2.5 x 2.5 mm |
| | Lattice Patterning | Direct patterning auxetic lattice with vertical disconnections |
| | Lattice Strut Diameter | 0.01 mm |
| | Outer Skirt (e.g., side wall 125) Thickness | 2 mm |
| | Outer Skirt Material | Dense |
| | Upper Endplate Interface Thickness | 1 mm |
| | Lower Endplate Thickness | 1 mm |
| Implant Material | 3D Printed Titanium Calibrated (Elastoplastic) | Johnson Cook |
| Inner Structure 124 | Lattice Beam Elements | 1D |
| | Lattice Beam per Strut | 1 |
| | Lattice Nodes Tetrahedral Solid Elements | 4 |
| | Lattice Solid Element Size | 0.1 mm to 0.3 mm |
| | Lattice Structure Element Type | 2-node beam with circular cross-section |
| Anatomical Parameters (FIG. 28) | Cortical Thickness | 0.80 mm (Anterior) 0.65 mm (Posterior) 0.6 mm (Upper Endplate) 0.45 mm (Lower Endplate) |
| | Endplate Lordosis Angle | 13.2° |
| | Anterior Height | 12.6 mm |
| | Biological Tissue | Linear elastic Tissue-specific Young's moduli |
| Boundary Conditions | Impulsion of sacrum | |

(continued)

|  | Lattice Structure Element Type | 2-node beam with circular cross-section |
|---|---|---|
|  | Node-surface penetration penalty interface with friction | Cage - endplates<br>Cage - intervertebral disc |
|  | Tied interface | Intervertebral disc - endplates |
|  | Degrees of freedom for sacrum | 1 (vertical translation) |
|  | Imposed load on L5 | 800 N |
|  | Imposed upwards displacement ramp on sacrum | 1 m/sec |
| Testing Conditions | Excitation Location | Base of Cage |
|  | Distal Displacement | +/- 0.45 mm |
|  | Resulting Distal Amplitude | 0.90 mm |
|  | Forced Excitation Frequency | 15 Hz |
| Observed Output | Von Mises stresses | FIGS. 29A-29C |
|  | Proximal amplitude | FIGS. 30-32 |

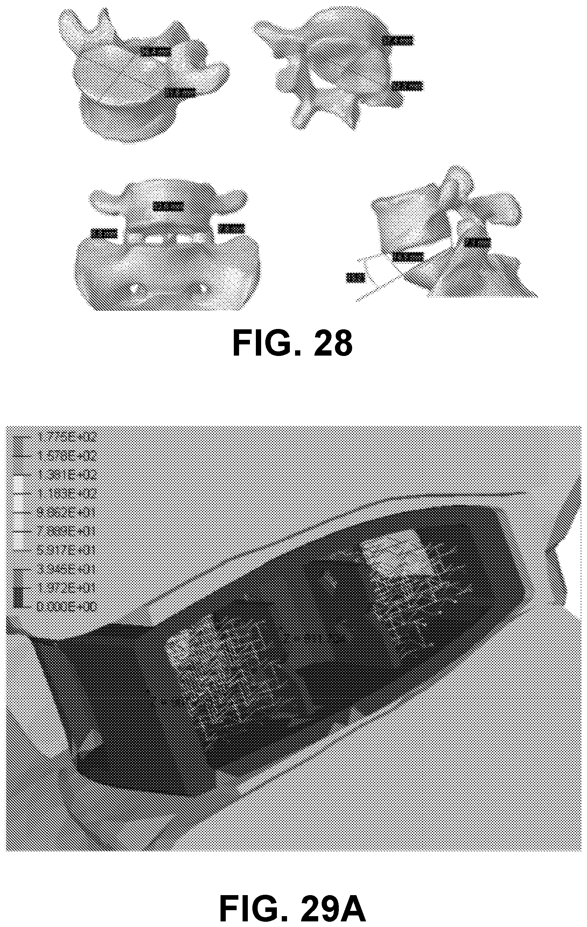

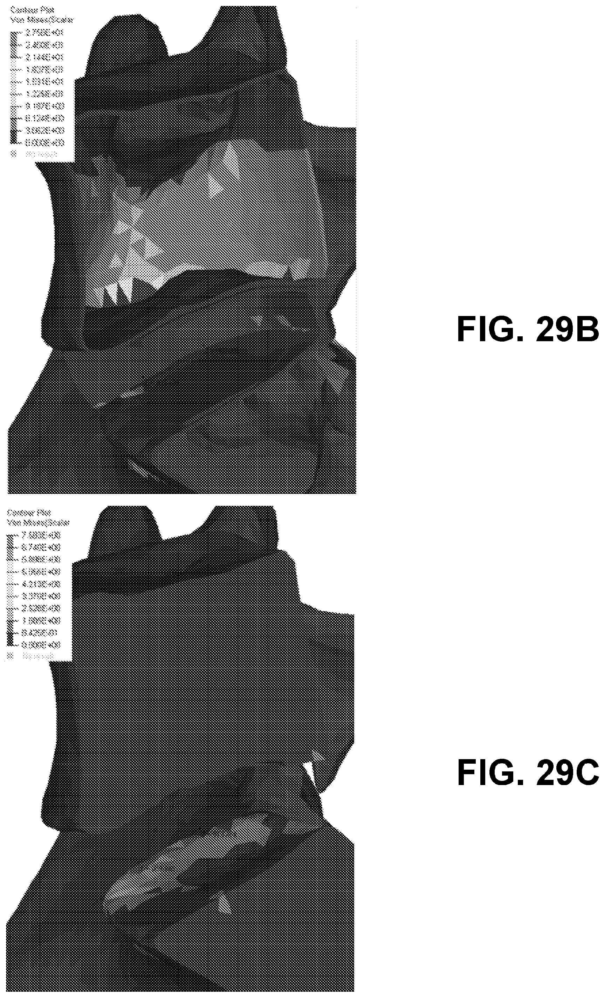

As shown in FIGS. 29A-29C, observed Von Mises stresses did not exceed the yield strength of the specified materials. The maximum stress in cortical bone was observed at 26.7 MPa, and 7.6 MPa in the trabecular bone, while the maximum stress in the endplates was 12.1 MPa. The maximum stress observed in the interbody cage portion 12 was observed to be 177.5 MPa.

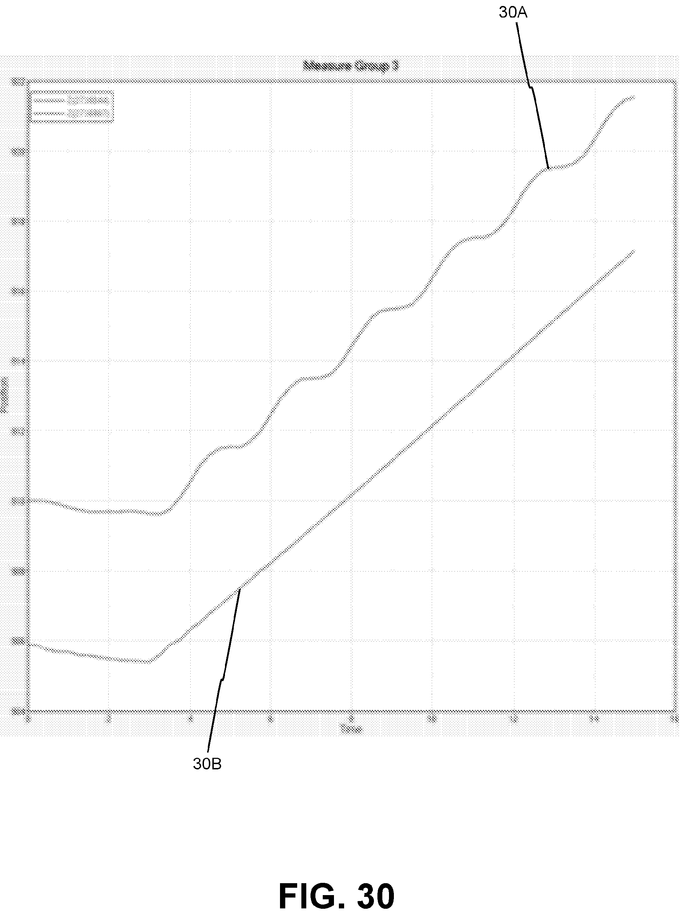

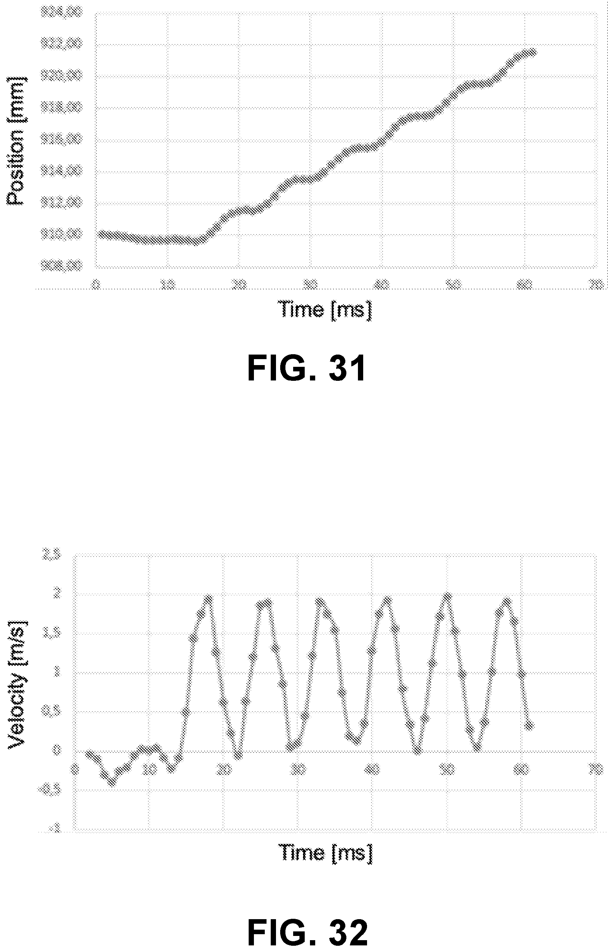

An oscillating motion was observed in the lattice structure 124 of the interbody cage portion 12, as shown in FIG. 30 (plot 30A) in response to the imposed displacement of the S1 spinal bone (plot 30B). Without wishing to be bound by theory, it is postulated that the observed oscillating motion may be due the lack of physical connection of the lattice structure 124 to the superior endplate 122a and the inferior endplate 122b. FIG. 31 shows the graft shield dynamic response as a function of position over time; FIG. 32 shows the graft shield dynamic response as a function of velocity over time.

Example 3: Modeling and Analysis of Free Resonators

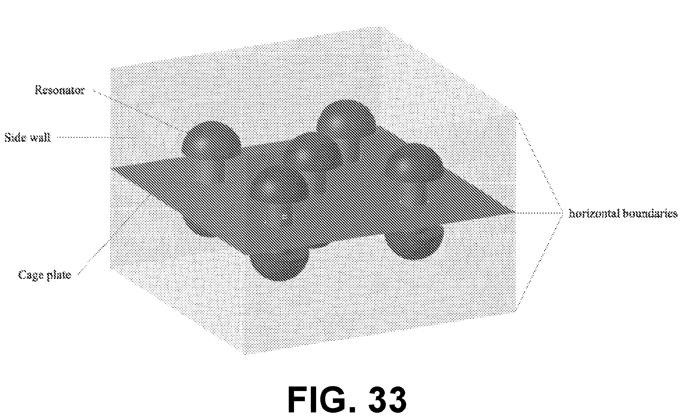

An *in silico* study of a dampening system including five free resonators 16 consistent with the embodiment shown in FIG. 23C was performed. The five free resonators 16 were oriented in parallel in a fine cage plate, surrounded by a bone graft cube obtained by a Boolean operation (FIG. 33). A fine element model simulating a forced excitation of 30 Hz and 90 Hz using a normal graft consistent with Simmons, Meguid & Pilliar (2001), and a softer graft representing fluid-like behavior before ossification. Relevant parameters are provided below.

| Free Resonator Geometry | Simple rod | 1.8 mm diameter |
|---|---|---|
|  | Mass at each extremity | 2 mm diameter |
|  | Cage plate holes | 2 mm diameter |
| Free Resonator Material | Titanium Elastoplastic |  |
| Graft Models | Soft graft material | Linear elastic |
|  | Soft graft E | 0.001 MPa |
|  | Normal graft model | 8 days post-operation interface zone tissue (Simmons, et al. (2001)) |
|  | Normal graft material | Linear elastic |
|  | Normal graft E | 1 MPa |
| Mesh Properties | Free Resonators | 0.4 mm tetrahedral elements |
|  | Cage Plate | 0.7 mm triangular elements |

(continued)

| | Graft | 0.4-1.7 mm tetrahedral elements |
|---|---|---|
| Boundary Conditions: Forced Vertical Excitation of Horizontal Boundaries | Displacement | +/- 0.2 mm |
| | Resulting Amplitude | 0.4 mm |
| | Frequencies | 30 Hz 90 Hz |
| Observed Output | Von Mises stresses in graft | FIGS. 34A-34C |
| | Dynamic Response of Free Resonator | FIGS. 35A-35C |
| | Maximum Amplitude of Dynamic Response | FIGS. 36A-36B |

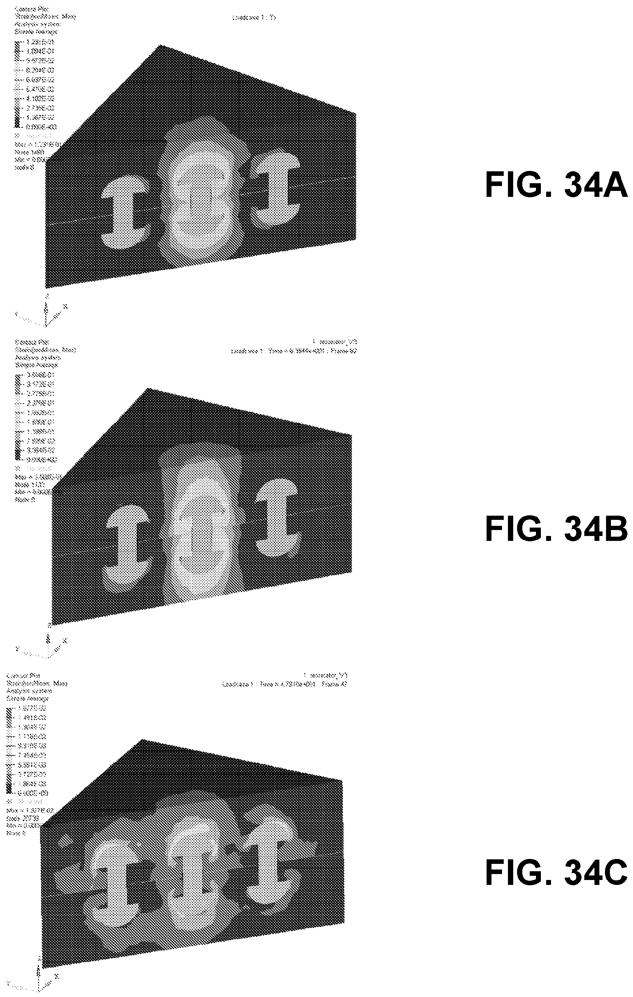

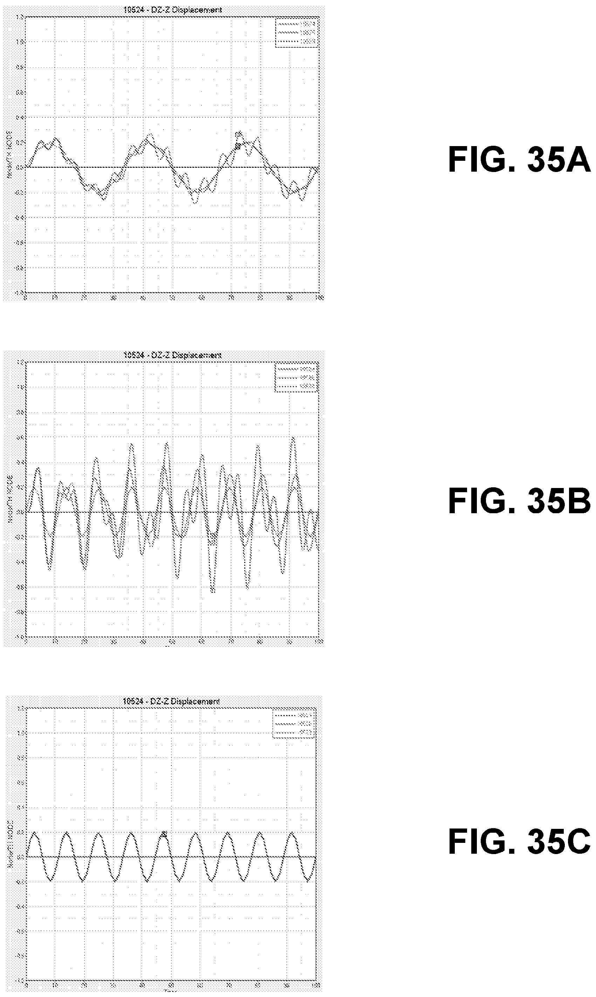

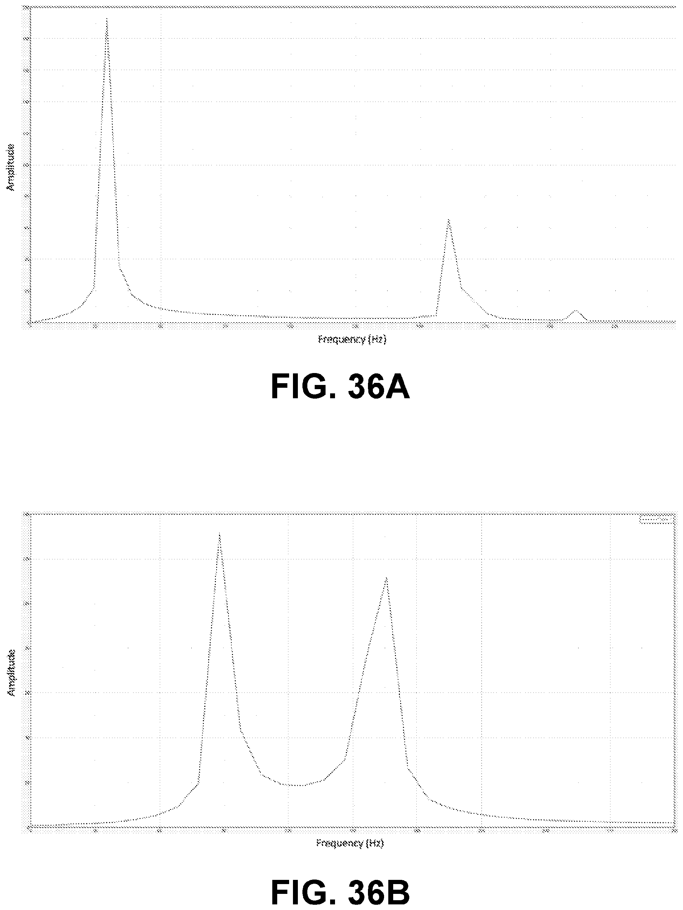

This study revealed that the central resonating element had a maximum amplitude of 0.57 mm in soft graft at 30Hz, an amplitude that was 42% higher than the input excitation (FIGS. 34A, 35A). As shown in FIG. 36A, a second mode at about 160 Hz emerged, suggesting that promotion of vibrations at specific frequencies might be possible using tuned resonating elements 16. The observed maximal tissue strain for this model was 12.3%, which is superior to the recommended maximal strain of 8% for ingrown bone (Lin et al. (2004)).

The central resonating element in soft graft responded even more unexpectedly at 90 Hz, with a maximum amplitude of 1.22 mm-more than twice the input amplitude of 0.4 mm (FIGS. 34B, 35B). A second mode at about 175 Hz was observed in this model (FIG. 36B), further suggesting that a tuned resonating element 16 may be employed to promote a desired vibrational frequency. Maximum tissue strain observed in this model was 35.7%, also superior to the prescribed 8% tissue strain level.

When this model was tested in normal graft at 90 Hz excitation, the dynamic response of the resonating element 16 was synchronized to the excitation (FIGS. 34C, 35C). The maximum amplitude was 0.412 mm, and the maximum graft strain was only 1.7% under these conditions. These data suggest that vibration isolation and promotion are unlikely to occur when graft transitions from soft graft to normal graft (e.g., many days after surgery).

## CONCLUSION

The foregoing descriptions of specific embodiments of the present invention have been presented for purposes of illustration and description. They are not intended to be exhaustive or to limit the invention to the precise forms disclosed, and obviously many modifications and variations are possible in light of the above teaching. The embodiments were chosen and described in order to best explain the principles of the invention and its practical application, to thereby enable others skilled in the art to best utilize the invention and various embodiments with various modifications as are suited to the particular use contemplated. It is intended that the scope of the invention be defined by the claims appended hereto.

It is to be understood that both the foregoing descriptions are exemplary and explanatory only, and are not restrictive of the methods and devices described herein. In this application, the use of the singular includes the plural unless specifically stated otherwise. Also, the use of "or" means "and/or" unless stated otherwise. Similarly, "comprise," "comprises," "comprising," "include," "includes" and "including" are not intended to be limiting.

## Claims

1. A dynamic spinal implant configured to be inserted between two adjacent vertebrae, the device comprising:

   a first endplate (122a) configured to contact the inferior face of a first vertebrae;
   a second endplate (122b) substantially opposite the first endplate and configured to contact the superior face of a second, adjacent vertebrae;
   an inner structure (124) disposed between the first endplate and the second endplate;
   and a bone graft or bone substitute material disposed within the dynamic spinal implant,
   wherein the inner structure is configured to generate a modified strain tensor on the bone graft and/or bone substitute material disposed within the dynamic spinal implant,
   wherein the inner structure is configured to modify a mechanical load to promote bone growth within the inner structure, and wherein the dynamic spinal implant comprises at least one resonating element (16).

2. The dynamic spinal implant of claim 1, wherein at least a portion of the inner structure includes a vibrational mode that resonates with the mechanical load.

3. The dynamic spinal implant of claim 1, wherein the inner structure is configured to focus the mechanical load toward or away from a predetermined location within the inner structure.

4. The dynamic spinal implant of any one preceding claim, wherein the dynamic spinal implant further comprises at least one free resonator (16).

5. The dynamic spinal implant of claim 1, wherein the dynamic spinal implant comprises a first resonating element having a first axis of motion, and either a second resonating element having a second axis of motion different from the first axis of motion, or a free resonator having a second axis of motion different than the first axis of motion.

6. The dynamic spinal implant of claim 1, wherein the inner structure comprises a plurality of orthorhombic body-centered repeating units (124a).

7. The dynamic spinal implant of any preceding claim, wherein the inner structure comprises at least one of:

    a plurality of dode-thin lattice repeating units,
    a plurality of diamond lattice repeating units,
    a plurality of auxetic lattice repeating units, or
    a plurality of S-shaped lattice repeating units.

8. The dynamic spinal implant of any preceding claim, wherein the strain tensor generated by the dynamic spinal implant causes fusion of the adjacent vertebrae faster than a static spinal implant.

9. The dynamic spinal implant of any preceding claim wherein, after implantation between two adjacent vertebrae of a subject, the dynamic spinal implant imparts a strain on surrounding tissue of at least about 8%.

10. The dynamic spinal implant of any preceding claim further comprising a side wall (125). disposed between the first endplate and the second endplate.

11. The dynamic spinal implant of claim 10 further comprising a slit (126) disposed on the side wall such that the dynamic spinal implant enables a strain tensor component along its z-axis that is greater than a strain tensor component enabled along its x-axis and that is greater than a strain tensor component enabled along its y-axis.

12. The dynamic spinal implant of claim 11, wherein the slit is disposed either proximal to a fixation area of the side wall, or proximal to an instrument interface area.

13. The dynamic spinal implant of any of claims 10-12, wherein the dynamic spinal implant resists compression along its z-axis at a first resistance level for a first compression distance along the z-axis, and resists compression along its z-axis at a second, greater resistance level for a second compression distance along the z-axis.

14. The dynamic spinal implant of any preceding claim, wherein the at least one resonating element is sized and shaped to vibrate at a desired frequency.

15. The dynamic spinal implant of claim 14, wherein the at least one resonating element comprises a mass associated with a stem portion.

16. The dynamic spinal implant of claim 15, wherein the at least one resonating element has a fundamental harmonic frequency of about 10 kHz to about 100 kHz.

17. The dynamic spinal implant of claim 15 or claim 16, wherein the at least one resonating element comprises one of: a disc-shaped mass portion disposed at a distal end of a stem portion, and a spherical mass portion disposed at a distal end of a stem portion.

**Patentansprüche**

1. Dynamisches Wirbelsäulenimplantat, das konfiguriert ist, um zwischen zwei benachbarten Wirbeln eingesetzt zu werden, wobei die Vorrichtung Folgendes umfasst:

   eine erste Endplatte (1228), die konfiguriert ist, um die untere Fläche eines ersten Wirbels zu berühren;
   eine zweite Endplatte (122b), die der ersten Endplatte im Wesentlichen gegenüberliegt und konfiguriert ist, um die obere Fläche eines zweiten benachbarten Wirbels zu berühren;
   eine innere Struktur (124), die zwischen der ersten Endplatte und der zweiten Endplatte angeordnet ist; und wobei die innere Struktur konfiguriert ist, um einen modifizierten Spannungstensor an dem Knochentransplantat und/oder Knochenersatzmaterial, das in dem dynamischen Wirbelsäulenimplantat angeordnet ist, zu erzeugen, wobei die innere Struktur konfiguriert ist, um eine mechanische Last zu modifizieren, um Knochenwachstum in der inneren Struktur zu fördern, und wobei das dynamische Wirbelsäulenimplantat mindestens ein Resonanzelement (16) umfasst.

2. Dynamisches Wirbelsäulenimplantat nach Anspruch 1, wobei mindestens ein Abschnitt der inneren Struktur einen Vibrationsmodus beinhaltet, der mit der mechanischen Last in Resonanz schwingt.

3. Dynamisches Wirbelsäulenimplantat nach Anspruch 1, wobei die innere Struktur konfiguriert ist, um die mechanische Last zu einer vorbestimmten Stelle in der inneren Struktur hin oder von dieser weg zu fokussieren.

4. Dynamisches Wirbelsäulenimplantat nach einem der vorhergehenden Ansprüche, wobei das dynamische Wirbelsäulenimplantat ferner mindestens einen freien Resonator (16) umfasst.

5. Dynamisches Wirbelsäulenimplantat nach Anspruch 1, wobei das dynamische Wirbelsäulenimplantat ein erstes Resonanzelement mit einer ersten Bewegungsachse und entweder ein zweites Resonanzelement mit einer zweiten Bewegungsachse, die sich von der ersten Bewegungsachse unterscheidet, oder einen freien Resonator mit einer zweiten Bewegungsachse, die sich von der ersten Bewegungsachse unterscheidet, umfasst.

6. Dynamisches Wirbelsäulenimplantat nach Anspruch 1, wobei die innere Struktur eine Vielzahl von orthorhombischen körperzentrierten Wiederholungseinheiten (124a) umfasst.

7. Dynamisches Wirbelsäulenimplantat nach einem der vorhergehenden Ansprüche, wobei die innere Struktur mindestens eines von Folgendem umfasst:

   eine Vielzahl von Dode-Thin-Gitter-Wiederholungseinheiten,
   eine Vielzahl von Diamant-Gitter-Wiederholungseinheiten,
   eine Vielzahl von auxetischen Gitter-Wiederholungseinheiten oder
   eine Vielzahl von S-förmigen Gitter-Wiederholungseinheiten.

8. Dynamisches Wirbelsäulenimplantat nach einem der vorhergehenden Ansprüche, wobei der durch das dynamische Wirbelsäulenimplantat erzeugte Spannungstensor eine Fusion der benachbarten Wirbel schneller als ein statisches Wirbelsäulenimplantat bewirkt.

9. Dynamisches Wirbelsäulenimplantat nach einem der vorhergehenden Ansprüche, wobei das dynamische Wirbelsäulenimplantat nach der Implantation zwischen zwei benachbarten Wirbeln eines Subjekts eine Spannung von mindestens etwa 8 % auf umgebendes Gewebe ausübt.

10. Dynamisches Wirbelsäulenimplantat nach einem der vorhergehenden Ansprüche, ferner umfassend eine Seitenwand (125), die zwischen der ersten Endplatte und der zweiten Endplatte angeordnet ist.

11. Dynamisches Wirbelsäulenimplantat nach Anspruch 10, ferner umfassend einen Schlitz (126), der an der Seitenwand derart angeordnet ist, dass das dynamische Wirbelsäulenimplantat eine Spannungstensorkomponente entlang seiner z-Achse ermöglicht, die größer als eine Spannungstensorkomponente ist, die entlang seiner x-Achse ermöglicht ist, und die größer als eine Spannungstensorkomponente ist, die entlang seiner y-Achse ermöglicht ist.

12. Dynamisches Wirbelsäulenimplantat nach Anspruch 11, wobei der Schlitz entweder proximal zu einem Befestigungsbereich der Seitenwand oder proximal zu einem Instrumentenschnittstellenbereich angeordnet ist.

**13.** Dynamisches Wirbelsäulenimplantat nach einem der Ansprüche 10-12,
wobei das dynamische Wirbelsäulenimplantat
Kompression entlang seiner z-Achse bei einem ersten Widerstandsniveau für eine erste Kompressionsdistanz entlang der z-Achse widersteht und Kompression entlang seiner z-Achse bei einem zweiten, größeren Widerstandsniveau für eine zweite Kompressionsdistanz entlang der z-Achse widersteht.

**14.** Dynamisches Wirbelsäulenimplantat nach einem der vorhergehenden Ansprüche, wobei das mindestens eine Resonanzelement bemessen und geformt ist, um bei einer gewünschten Frequenz zu vibrieren.

**15.** Dynamisches Wirbelsäulenimplantat nach Anspruch 14, wobei das mindestens eine Resonanzelement eine Masse umfasst, die einem Schaftabschnitt zugeordnet ist.

**16.** Dynamisches Wirbelsäulenimplantat nach Anspruch 15, wobei das mindestens eine Resonanzelement eine harmonische Grundfrequenz von etwa 10 kHz bis etwa 100 kHz aufweist.

**17.** Dynamisches Wirbelsäulenimplantat nach Anspruch 15 oder Anspruch 16, wobei das mindestens eine Resonanzelement eines der Folgenden umfasst:
einen scheibenförmigen Masseabschnitt, der an einem distalen Ende eines Schaftabschnitts angeordnet ist, und
einen kugelförmigen Masseabschnitt, der an einem distalen Ende eines Schaftabschnitts angeordnet ist.

**Revendications**

**1.** Implant vertébral dynamique configuré pour être inséré entre deux vertèbres adjacentes, le dispositif comprenant :

une première plaque d'extrémité (122a) configurée pour entrer en contact avec la face inférieure d'une première vertèbre ;
une deuxième plaque d'extrémité (122b) sensiblement opposée à la première plaque d'extrémité et configurée pour entrer en contact avec la face supérieure d'une deuxième vertèbre adjacente ;
une structure interne (124) disposée entre la première plaque d'extrémité et la deuxième plaque d'extrémité ;
et un greffon osseux ou un matériau de substitution osseuse disposé à l'intérieur de l'implant vertébral dynamique,
la structure interne est configurée pour générer un tenseur de contrainte modifié sur le greffon osseux et/ou le matériau de substitution osseuse disposé à l'intérieur de l'implant vertébral dynamique,
dans lequel la structure interne est configurée pour modifier une charge mécanique afin de favoriser la croissance osseuse dans la structure interne, et dans lequel l'implant vertébral dynamique comprend au moins un élément de résonance (16).

**2.** Implant vertébral dynamique de la revendication 1, dans lequel au moins une partie de la structure interne inclut un mode vibratoire qui résonne avec la charge mécanique.

**3.** Implant vertébral dynamique de la revendication 1, dans lequel la structure interne est configurée pour concentrer la charge mécanique vers ou à partir d'un emplacement prédéterminé à l'intérieur de la structure interne.

**4.** Implant vertébral dynamique de l'une des revendications précédentes, dans lequel l'implant vertébral dynamique comprend en outre au moins un résonateur libre (16).

**5.** Implant vertébral dynamique de la revendication 1, dans lequel l'implant vertébral dynamique comprend un premier élément de résonance ayant un premier axe de mouvement, et soit un deuxième élément de résonance ayant un deuxième axe de mouvement différent du premier axe de mouvement, soit un résonateur libre ayant un deuxième axe de mouvement différent du premier axe de mouvement.

**6.** Implant spinal dynamique de la revendication 1, dans lequel la structure interne comprend une pluralité d'unités répétitives orthorhombiques centrées sur le corps (124a).

**7.** Implant spinal dynamique de toute revendication précédente, dans lequel la structure interne

comprend au moins l'un des éléments suivants

une pluralité d'unités répétitives de treillis dodécagonal,
une pluralité d'unités répétitives du réseau de diamants,
une pluralité d'unités répétitives de treillis auxétiques, ou
une pluralité d'unités répétitives en treillis en forme de S.

8. Implant vertébral dynamique de toute revendication précédente, dans lequel le tenseur de contrainte généré par l'implant vertébral dynamique provoque la fusion des vertèbres adjacentes plus rapidement qu'un implant vertébral statique.

9. Implant vertébral dynamique de toute revendication précédente dans lequel, après implantation entre deux vertèbres adjacentes d'un sujet, l'implant vertébral dynamique transmet une contrainte sur le tissu environnant d'au moins 8 % environ.

10. Implant spinal dynamique de toute revendication précédente comprend en outre une paroi latérale (125) disposée entre la première plaque d'extrémité et la deuxième plaque d'extrémité.

11. Implant vertébral dynamique de la revendication 10 comprend en outre une fente (126) disposée sur la paroi latérale de sorte que l'implant vertébral dynamique active une composante du tenseur de contrainte le long de son axe z qui est supérieure à une composante du tenseur de contrainte activée le long de son axe x et qui est supérieure à une composante du tenseur de contrainte activée le long de son axe y.

12. Implant vertébral dynamique de la revendication 11, dans lequel la fente est disposée soit à proximité d'une zone de fixation de la paroi latérale, soit à proximité d'une zone d'interface avec l'instrument.

13. Implant spinal dynamique de l'une des revendications 10 à 12,
dans lequel l'implant vertébral dynamique résiste à la compression le long de son axe z à un premier niveau de résistance pour une première distance de compression le long de l'axe z, et résiste à la compression le long de son axe z à un deuxième niveau de résistance plus important pour une deuxième distance de compression le long de l'axe z.

14. Implant spinal dynamique de toute revendication précédente, dans lequel l'au moins un élément de résonance est dimensionné et formé pour vibrer à une fréquence souhaitée.

15. Implant vertébral dynamique de la revendication 14, dans lequel l'au moins un élément de résonance comprend une masse associée à une partie de la tige.

16. Implant spinal dynamique de la revendication 15, dans lequel l'au moins un élément résonant a une fréquence harmonique fondamentale d'environ 10 kHz à environ 100 kHz.

17. Implant spinal dynamique de la revendication 15 ou de la revendication 16, dans lequel l'au moins un élément de résonance comprend l'un des éléments suivants :
une partie de masse en forme de disque disposée à l'extrémité distale d'une partie de la tige, et une partie de masse sphérique disposée à l'extrémité distale d'une partie de la tige.

**FIG. 1**

**FIG. 2A**

**FIG. 2B**

**FIG. 3A**

**FIG. 3B**

**FIG. 3C**

**FIG. 4**

**FIG. 5**

**FIG. 6**

124a →

W

H

L

124s

124d

129

**FIG. 7**

124a →

W

H

L

124s

124d

**FIG. 8A**

124f

124a →

124f

129

124s

124d

124f'

**FIG. 8B**

124fd

124f

FIG. 9

124a →

W    L

H

124s

129

124d

124d'

FIG. 10

16 →

162d

162

164a

164

164d

164b

162h

164L

FIG. 11

16 →

162

162r

164a

164

164d

164L

164b

FIG. 12

16

124s/124f/124c

162W

162

164

164a

124t

164d

124W    124td

164L

162'

164b

162'W

**FIG. 13**

**FIG. 14**

**FIG. 15**

**FIG. 16**

**FIG. 17**

**FIG. 18**

**FIG. 19A**

**FIG. 19B**

125  BG  123  123p

12

FIG. 20

12

125'
125'
12'a
12'a
125

FIG. 21A

12'

12

12'
125
125'

FIG. 21B

**FIG. 22**

**FIG. 23A**

**FIG. 23B**

**FIG. 23C**

**FIG. 24A**

**FIG. 24B**

**FIG. 25A**

**FIG. 25B**

12

125

124

127

# FIG. 26

**FIG. 27**

FIG. 28

FIG. 29A

FIG. 29B

FIG. 29C

**FIG. 30**

**FIG. 31**

**FIG. 32**

Resonator

Side wall

Cage plate

horizontal boundaries

# FIG. 33

FIG. 34A

FIG. 34B

FIG. 34C

**FIG. 35A**

**FIG. 35B**

**FIG. 35C**

**FIG. 36A**

**FIG. 36B**

12 ➔

124

# FIG. 37A

12 ➔

124

# FIG. 37B

**FIG. 38A**

**FIG. 38B**

**FIG. 38C**

**FIG. 38D**

FIG. 38E

FIG. 38F

FIG. 38G

FIG. 38H

**FIG. 38I**

**FIG. 38J**

EP 4 161 450 B1

12

125

125

126'

**FIG. 38K**

12

127a

124

122a

**FIG. 38L**

50

**FIG. 39**

**FIG. 40**

**FIG. 41A**

**FIG. 41B**

**FIG. 41C**

FIG. 42

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 20180280145 A